(19) Europäisches Patentamt / European Patent Office / Office européen des brevets

(11) **EP 2 829 864 B1**

(12) **EUROPEAN PATENT SPECIFICATION**

(45) Date of publication and mention
of the grant of the patent:
**18.10.2017 Bulletin 2017/42**

(51) Int Cl.:
***G01N 21/27*** (2006.01)    ***F16H 57/04*** (2010.01)
***G01N 33/30*** (2006.01)

(21) Application number: **13764146.0**

(22) Date of filing: **19.03.2013**

(86) International application number:
**PCT/JP2013/057898**

(87) International publication number:
**WO 2013/141260 (26.09.2013 Gazette 2013/39)**

(54) **SPEED REDUCER BREAKAGE STATE-INFORMING DEVICE, MACHINE SYSTEM WITH SPEED REDUCER BREAKAGE STATE-INFORMING FUNCTION, AND MEDIUM HAVING SPEED REDUCER BREAKAGE STATE-INFORMING PROGRAM RECORDED THEREON**

VORRICHTUNG ZUR INFORMATION ÜBER DEN BRUCHSTATUS EINES UNTERSETZUNGSGETRIEBES, MASCHINENSYSTEM MIT FUNKTION ZUR INFORMATION ÜBER DEN BRUCHSTATUS EINES UNTERSETZUNGSGETRIEBES UND MEDIUM MIT EINEM DARAUF AUFGEZEICHNETEN PROGRAMM ZUR INFORMATION ÜBER DEN BRUCHSTATUS EINES UNTERSETZUNGSGETRIEBES

DISPOSITIF D'INFORMATION D'ÉTAT DE RUPTURE DE RÉDUCTEUR DE VITESSE, SYSTÈME DE MACHINE AVEC FONCTION D'INFORMATION D'ÉTAT DE RUPTURE DE RÉDUCTEUR DE VITESSE, ET SUPPORT AYANT UN PROGRAMME D'INFORMATION D'ÉTAT DE RUPTURE DE RÉDUCTEUR DE VITESSE ENREGISTRÉ DESSUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.03.2012 JP 2012062058**

(43) Date of publication of application:
**28.01.2015 Bulletin 2015/05**

(73) Proprietor: **Nabtesco Corporation
Tokyo 102-0093 (JP)**

(72) Inventors:
• **NAKAMURA, Koji
Tsu-shi, Mie 514-8533 (JP)**
• **SHIMADA, Hideshi
Tsu-shi, Mie 514-8533 (JP)**

(74) Representative: **Grünecker Patent- und Rechtsanwälte
PartG mbB
Leopoldstraße 4
80802 München (DE)**

(56) References cited:
EP-A1- 0 590 487          WO-A1-2008/034945
WO-A1-2012/074112    JP-A- H07 146 233
JP-A- 2000 240 478      JP-A- 2002 188 649
JP-A- 2007 192 769      JP-A- 2007 212 161
JP-A- 2008 020 327      JP-U- H0 635 984
US-A1- 2008 024 761

# Description

The present invention relates to a reducer damage-state notification apparatus for notifying the damage state of the reducer of a machine which includes the reducer and a lubricant deterioration sensor for detecting the deterioration of lubricant of the reducer.

Conventionally, as a lubricant deterioration sensor for detecting the deterioration of lubricant of a machine, an oil deterioration degree sensor has been known. In this sensor, an oil entering gap portion for entering lubricant therein is formed on an optical path from an infrared LED (Light Emitting Diode) to a photo diode. An amount of light absorbed by the lubricant within the oil entering gap portion, with respect to light emitted from the infrared LED, is measured according to a light reception amount of the photo diode. Then, a deterioration degree of the lubricant related to the light absorption amount thus measured is determined (see patent literatures 1 and 2, for example). A further example for monitoring oil deterioration based on light passing through the oil medium is described in patent literature 3.

However, the oil deterioration degree sensor described in each of the patent literatures 1 and 2 has a problem that, although a density of insoluble content within the lubricant can be measured as the deterioration degree of the lubricant, kinds of contaminant within the lubricant cannot be specified.

As a technique of specifying the kinds of contaminant within lubricant, there has been known a technique in which an LED irradiates light toward a membrane filter having been used to filter the lubricant. Then, a light reception element converts reflection light from the contaminant on the membrane filter into digital values of RGB, to thereby specify the kinds of contaminant within the lubricant based on the RGB digital values thus converted (see non-patent literatures 1 and 2, for example).

> PATENT LITERATURE 1: JP-A-7-146233
> PATENT LITERATURE 2: JP-A-10-104160 Closest prior document: US 2008/024761 A1
> NON-PATENT LITERATURE 1: Tomohiko Yamaguchi and four others, "METHOD OF DISCRIMINATING HUE OF CONTAMINANT WITHIN LUBRICANT", Fukui University, Faculty of Engineering, Research Report, March 2003, Volume 51, No. 1, pp. 81 - 88
> NON-PATENT LITERATURE 2: Tomomi Honda, "DETERIORATION DIAGNOSIS OF LUBRICANT •INSPECTION TECHNOLOGY", Journal of Japan Society for Precision Engineering, 2009, Volume 75, No. 3, pp. 359 - 362

Since the technique described in each of the non-patent literatures 1 and 2 requires draining the lubricant from a machine and filtering the lubricant by a membrane filter, there arises a problem of lacking immediacy.

Thus, the inventors of the present application have developed a lubricant deterioration sensor which is provided in a machine including a reducer and enables to immediately specify the kinds and amounts of contaminant within lubricant of the reducer. The lubricant deterioration sensor (hereinafter referred to "new sensor") includes a light emitting element which emits light, a color light reception element which detects colors of received light, and a gap forming member which is formed with an oil gap disposed on an optical path from the light emitting element to the color light reception element so as to transmit light therethrough. The oil gap acts as a gap for entering the lubricant of the reducer therein.

The inventors of the present application have developed a reducer damage-state notification apparatus which notifies a damage state of the reducer corresponding to a predetermined threshold, when a color difference between the colors detected by the color light reception element of the new sensor and a predetermined color reaches the threshold.

However, the inventors of the present application have discovered that a relation between the color difference, which is a difference between the colors detected by the color light reception element of the new sensor and the predetermined color, and the damage state of the reducer differs depending on the working condition of the reducer such as temperature of the lubricant. When the relation between the color difference, which is the difference between the colors detected by the color light reception element of the new sensor and the predetermined color, and the damage state of the reducer is not constant, there arises a problem that the reducer damage-state notification apparatus cannot notify the damage state of the reducer accurately.

An object of the invention is to provide a reducer damage-state notification apparatus which can notify the damage state of a reducer accurately even when the working condition of the reducer differs.

This is achieved by the features of the independent claims.

The reducer damage-state notification apparatus sets the threshold for notifying the damage state of the reducer according to the temperature of the lubricant. Thus, even in a case that the temperature of the lubricant as the working condition of the reducer is different, the damage state of the reducer can be notified accurately.

The reducer damage-state notification apparatus sets the threshold for notifying the damage state of the reducer according to the actual temperature of the lubricant. Thus, the damage state of the reducer can be notified accurately in accordance with the actual temperature of the lubricant.

The reducer damage-state notification apparatus sets the threshold for notifying the damage state of the reducer according to the average value of the actual temperature of the lubricant during the predetermined time period. Thus, even in a case that the temperature of the lubricant rapidly changes temporarily, erroneous notification of the damage state of the reducer can be prevented.

In such a case just after the exchange of the lubricant for new one, the lubricant may not deteriorate immediately even when the reducer approaches actually to a damaged state or has been already damaged. When the lubricant is not deteriorated yet, the color difference between the color detected by the color light reception element and the predetermined color does not reach the threshold. However, when the reducer approaches actually to a damaged state or has been already damaged, since the reducer intensely generates heat, the temperature of the lubricant increases rapidly. The reducer damage-state notification apparatus according to the invention notifies the damage state of the reducer in the case where the increasing amount of the average value of the actual temperature of the lubricant during the predetermined time period is the predetermined value or more. Thus, when the reducer approaches actually to a damaged state or has been already damaged, even if the color difference between the color detected by the color light reception element and the predetermined color does not reach the threshold, the damage state of the reducer can be notified suitably.

The reducer damage-state notification apparatus according to the invention sets the threshold for notifying the damage state of the reducer according to the temperature of the lubricant and the oil sealing rate. Thus, even in the case that the temperature of the lubricant and the oil sealing rate as the working condition of the reducer are different, the damage state of the reducer can be notified accurately.

The reducer damage-state notification apparatus according to the invention sets the threshold for notifying the damage state of the reducer according to the oil sealing rate. Thus, even in the case that the oil sealing rate as the working condition of the reducer is different, the damage state of the reducer can be notified accurately.

The reducer damage-state notification apparatus according to the invention can set the threshold for notifying the damage state of the reducer according to the oil sealing rate and the type of the reducer by merely inputting the amount of the lubricant and the type of the reducer.

The reducer damage-state notification apparatus according to the invention sets the threshold for notifying the damage state of the reducer according to the capacity of the oil accommodation space. Thus, even in the case that the capacity of the oil accommodation space as the working condition of the reducer is different, the damage state of the reducer can be notified accurately.

The reducer damage-state notification apparatus according to the invention can notify the damage state of the reducer as plural levels of the probability of the damage of the reducer.

The machine system with reducer damage-state notification function according to the invention can notify the damage state of the reducer accurately even in a case that the working condition of the reducer is different.

The machine system with reducer damage-state notification function according to the invention can notify the damage state of the reducer accurately even in a case that the working condition of the reducer is different. Further, since the lubricant temperature sensor is contained in the lubricant deterioration sensor, the machine system with reducer damage-state notification function according to the invention can be configured easily as compared with a configuration where the lubricant temperature sensor is provided separately from the lubricant deterioration sensor.

The computer for executing the reducer damage-state notification program according to the invention can notify the damage state of the reducer accurately even in a case that the working condition of the reducer is different.

As explained above, the reducer damage-state notification apparatus according to the invention can notify the damage state of the reducer accurately even in a case that the working condition of the reducer is different.

[Fig. 1] Fig. 1 is a block diagram showing a machine system with reducer damage-state notification function according to an embodiment of the invention.

[Fig. 2] Fig. 2 is a side view of an industrial robot shown in Fig. 1.

[Fig. 3] Fig. 3 is a sectional view of the articular portion of the industrial robot shown in Fig. 1.

[Fig. 4] Fig. 4 is a front view of a lubricant deterioration sensor shown in Fig. 3.

[Fig. 5] Fig. 5 is a front sectional view of the lubricant deterioration sensor shown in Fig. 4 in an attached state to an arm.

[Fig. 6] Fig. 6(a) is a plan view of the lubricant deterioration sensor shown in Fig. 4. Fig. 6(b) is a bottom view of the lubricant deterioration sensor shown in Fig. 4.

[Fig. 7] Fig. 7 is a diagram showing an optical path from a white LED to an RGB sensor shown in Fig. 5.

[Fig. 8] Fig. 8 is a block diagram showing a reducer damage-state notification apparatus shown in Fig. 1.

[Fig. 9] Fig. 9 is a diagram showing an example of a type/capacity correspondence table shown in Fig. 8.

[Fig. 10] Fig. 10 is a diagram showing an example of a threshold setting table shown in Fig. 8.

[Fig. 11] Fig. 11 is a diagram showing an example of a table selection table shown in Fig. 8.

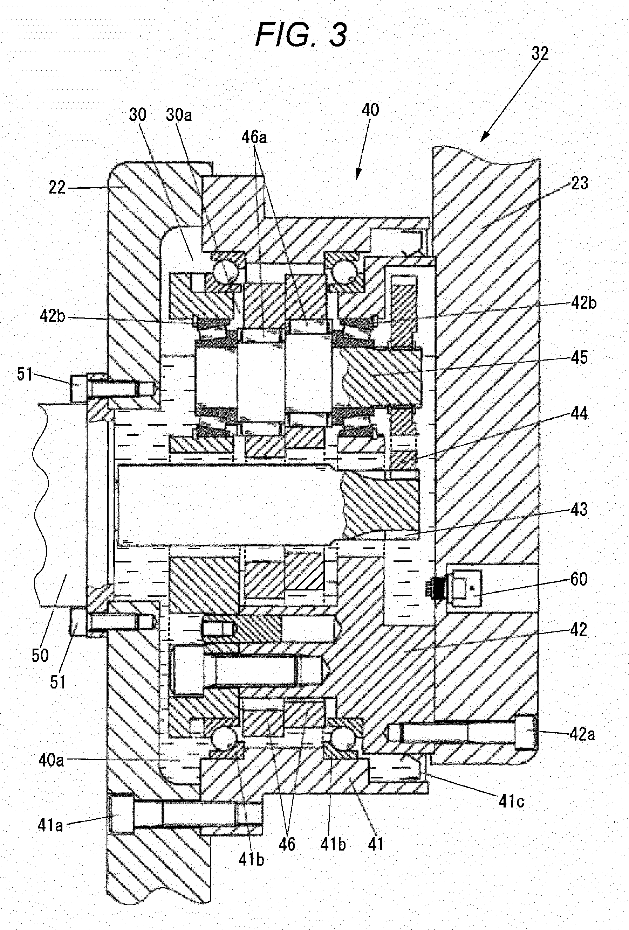

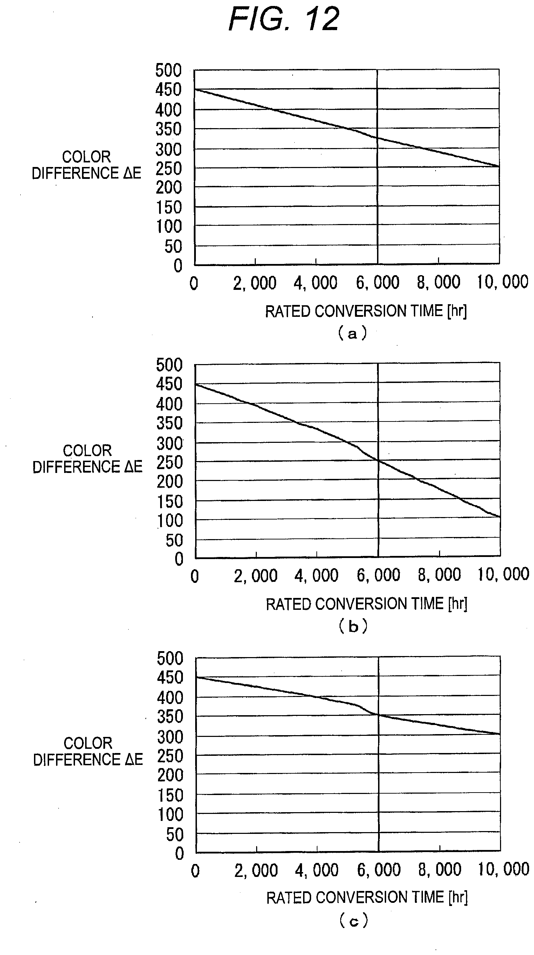

[Fig. 12] Fig. 12(a) is a graph showing an example of the experimentation result of the chronological change of a color difference ΔE in a case where the temperature of lubricant shown in Fig. 3 is 40°C and an oil sealing rate is 80%. Fig. 12(b) is a graph showing an example of the experimentation result of the chronological change of the color difference ΔE in a case where the temperature of lubricant shown in Fig. 3 is 60°C and the oil sealing rate is 80%. Fig. 12(c) is a graph showing an example of the experimentation result of the chronological change of a color difference ΔE in a case where the temperature of lubricant shown in Fig. 3 is 60°C and the oil sealing rate is 110%.

[Fig. 13] Fig. 13 is a flowchart showing the operation of the reducer damage-state notification apparatus shown in Fig. 8.

[Fig. 14] Fig. 14 is a flowchart showing the succeeding operation of Fig. 13.

[Fig. 15] Fig. 15 is a diagram showing an example of an information input screen displayed on a display unit shown in Fig. 8.

[Fig. 16] Fig. 16(a) is a diagram showing an example of a screen, to be displayed on the display unit shown in Fig. 8, for displaying a notification warning that the reducer is damaged with high probability. Fig. 16(b) is a diagram showing an example of the screen, to be displayed on the display unit shown in Fig. 8, for displaying a notification warning that the reducer may be damaged. Fig. 16(c) is a diagram showing an example of the screen, to be displayed on the display unit shown in Fig. 8, for displaying a notification promoting checking or repairing of the reducer to a user.

[Fig. 17] Fig. 17(a) is a schematic diagram of an articular portion shown in Fig. 3 in a case that the capacity of an intra-articular portion oil-accommodation space, the capacity of an intra-reducer oil-accommodation space, and the amount of the lubricant are 200 ml, 150 ml and 120 ml, respectively. Fig. 17(b) is a schematic diagram of the articular portion shown in Fig. 3 in a case that the capacity of the intra-articular portion oil-accommodation space, the capacity of the intra-reducer oil-accommodation space, and the amount of the lubricant are 300 ml, 150 ml and 120 ml, respectively.

Hereinafter, an embodiment of this invention will be explained with reference to drawings.

First, the explanation will be made as to the configuration of a machine system with reducer damage-state notification function according to the embodiment.

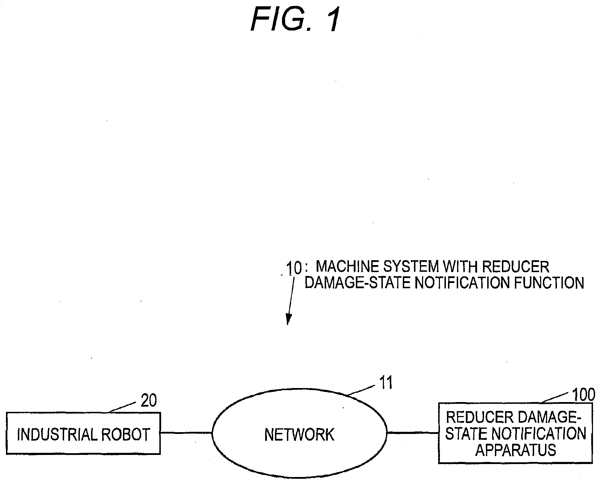

Fig. 1 is a block diagram showing the machine system 10 with reducer damage-state notification function according to the embodiment.

As shown in Fig. 1, the machine system 10 with reducer damage-state notification function includes an industrial robot 20 as a machine of the invention and a reducer damage-state notification apparatus 100 for notifying the damage state of the reducer of the industrial robot 20. The industrial robot 20 and the reducer damage-state notification apparatus 100 are mutually connected via a network 11 such as an LAN (Local Area Network) so as to be able to communicate to each other.

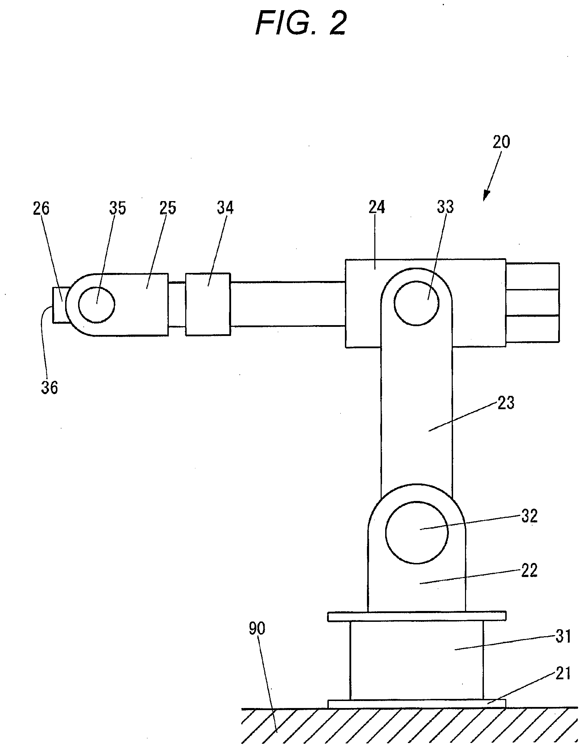

Fig. 2 is a side view of the industrial robot 20.

As shown in Fig. 2, the industrial robot 20 includes an attachment portion 21 to be attached to an installation placing portion 90 such as a floor or a ceiling; arms 22, 23, 24, 25 and 26; an articular portion 31 for connecting between the attachment portion 21 and the arm 22; an articular portion 32 for connecting between the arm 22 and the arm 23; an articular portion 33 for connecting between the arm 23 and the arm 24; an articular portion 34 for connecting between the arm 24 and the arm 25; an articular portion 35 for connecting between the arm 25 and the arm 26; and an articular portion 36 for connecting between the arm 26 and a not-shown hand.

Fig. 3 is a sectional view of the articular portion 32. Hereinafter, although the explanation is made as to the articular portion 32, each of the articular portions 31 and 33 to 36 is substantially same as this articular portion.

As shown in Fig. 3, the articular portion 32 includes a reducer 40 for connecting between the arm 22 and the arm 23, a motor 50 fixed to the arm 22 by means of bolts 51, and a lubricant deterioration sensor 60 for detecting the deterioration of lubricant 40a for reducing friction generated at the movable portions of the reducer 40.

In the articular portion 32, an intra-articular portion oil-accommodation space 30 as an oil accommodation space capable of accommodating the lubricant 40a is formed by the arms 22, 23 and the reducer 40. In the reducer 40, an intra-reducer oil-accommodation space 30a is formed as a part of the intra-articular portion oil-accommodation space 30.

The reducer 40 includes a case 41 fixed to the arm 22 by means of bolts 41a; a supporting body 42 fixed to the arm 23 by means of bolts 42a, a gear 43 fixed to the output shaft of the motor 50; three gears 44 which are disposed around the center axis of the reducer 40 with a constant interval therebetween and mesh with the gear 43; three crank shafts 45 which are disposed around the center axis of the reducer 40 with a constant interval therebetween and fixed to the gears 44, respectively; and two external gears 46 which mesh with internal gears provided at the case 41.

The supporting body 42 is rotatably supported by the case 41 via bearings 41b. A seal member 41c for preventing leakage of the lubricant 40a is provided between the case 41 and the supporting body 42.

Each of the crank shafts 45 is rotatably supported by the supporting body 42 via bearings 42b and further rotatably supported by the external gears 46 via bearings 46a.

The lubricant deterioration sensor 60 is fixed to the arm 23.

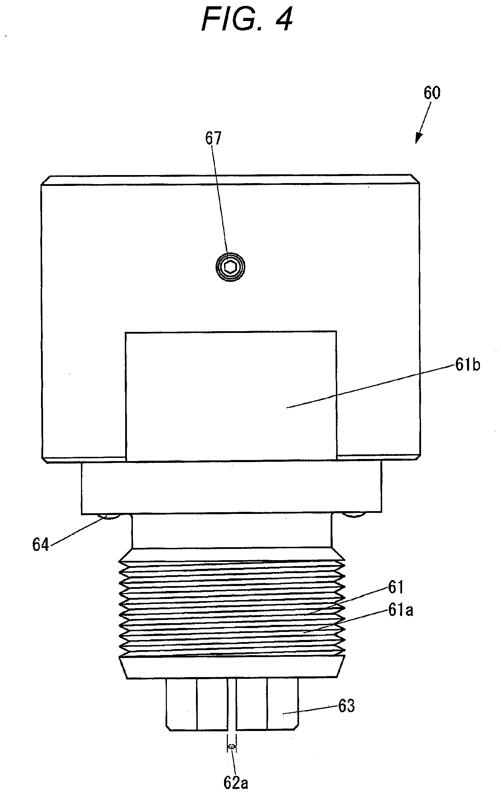

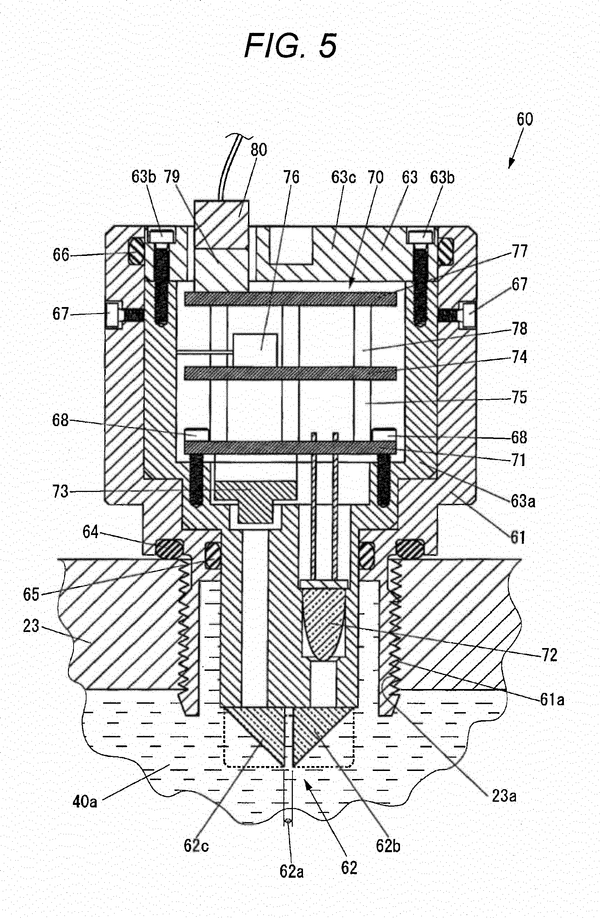

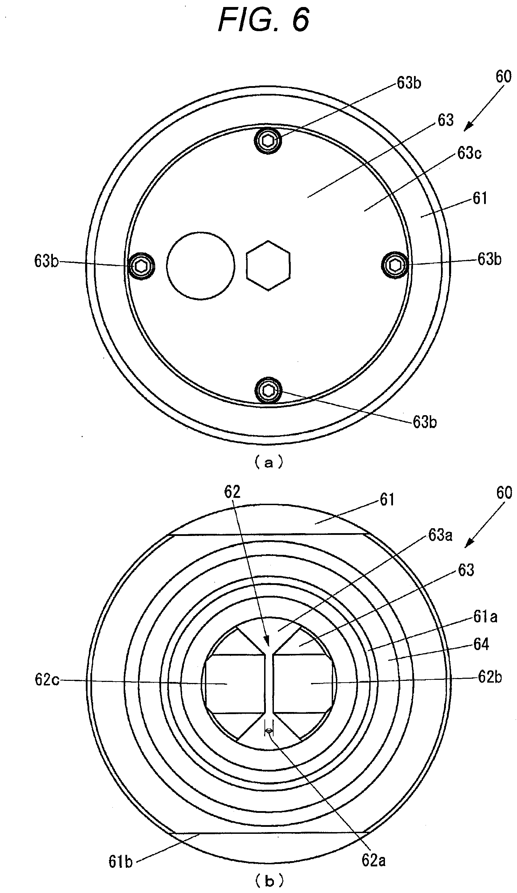

Fig. 4 is a front view of the lubricant deterioration sensor 60. Fig. 5 is a front sectional view of the lubricant deterioration sensor 60 in an attached state to the arm 23. Fig. 6(a) is a plan view of the lubricant deterioration sensor 60. Fig. 6(b) is a bottom view of the lubricant deterioration sensor 60.

As shown in Fig. 4 to Fig. 6(b), the lubricant deterioration sensor 60 includes a housing 61 made of aluminum alloy for supporting the respective components of the lubricant deterioration sensor 60; a gap forming member 62 forming an oil gap 62a into which the lubricant 40a enters; a supporting member 63 for supporting the gap forming member 62; an O ring 64 for preventing the leakage of the lubricant 40a from a gap between the housing 61 and the arm 23; an O ring 65 for preventing the leakage of the lubricant 40a from a gap between the housing 61 and the supporting member 63; an O ring 66 disposed between the housing 61 and the supporting member 63; and an electronic component group 70.

The housing 61 includes a screw portion 61a to be fixed within the screw hole 23a of the arm 23, and a tool contact portion 61b which is grasped by a tool such as a wrench at the time of rotating the screw portion 61a with respect to the screw hole 23a of the arm 23. The screw hole 23a of the arm 23 may be used for supplying the lubricant 40a to the reducer 40 and for disposing the lubricant 40a from the reducer 40, in a state that the lubricant deterioration sensor 60 is removed.

The gap forming member 62 is constituted by two right-angle prisms 62b, 62c made of glass. The oil gap 62a, as a gap for entering the lubricant 40a therein, is formed between the two right-angle prisms 62b, 62c. Each of the right-angle prisms 62b, 62c is fixed to the supporting member 63 by means of adhesive.

The supporting member 63 is fixed to the housing 61 by means of bolts 67 with hexagon holes. The supporting member 63 includes a holder 63a made of aluminum alloy, and a holder cap 63c made of aluminum alloy which is fixed to the holder 63a by means of bolts 63b with hexagon holes.

The electronic component group 70 includes a circuit board 71 fixed to the supporting member 63 by means of screws 68; a white LED 72 which is a light emitting element emitting white light and mounted on the circuit board 71; an RGB sensor 73 which is a color light reception element for detecting colors of received light and mounted on the circuit board 71; a circuit board 74 disposed on one surface side of the circuit board 71 opposite to the white LED 72 and RGB sensor 73 side thereof; a plurality of pillars 75 for fixing the circuit board 71 and the circuit board 74; a temperature sensor 76 which is a lubricant temperature sensor for detecting the temperature of the lubricant 40a via the holder 63a and mounted on the circuit board 74; a circuit board 77 disposed on one surface side of the circuit board 74 opposite to the circuit board 71 side thereof; a plurality of pillars 78 for fixing the circuit board 74 and the circuit board 77; and a connector 79 mounted on one surface side of the circuit board 77 opposite to the circuit board 74 side thereof. A plurality of electronic components are mounted on each of the circuit board 71, the circuit board 74 and the circuit board 77. The circuit board 71, the circuit board 74 and the circuit board 77 are mutually connected electrically.

The connector 79 is configured so that this connector is connected to the connector 80 of an external device of the lubricant deterioration sensor 60 so as to be supplied with electric power via the connector 80 from the external device, and the detection results of the RGB sensor 73 and the temperature sensor 76 is outputted respectively as electric signals to the external device via the connector 80.

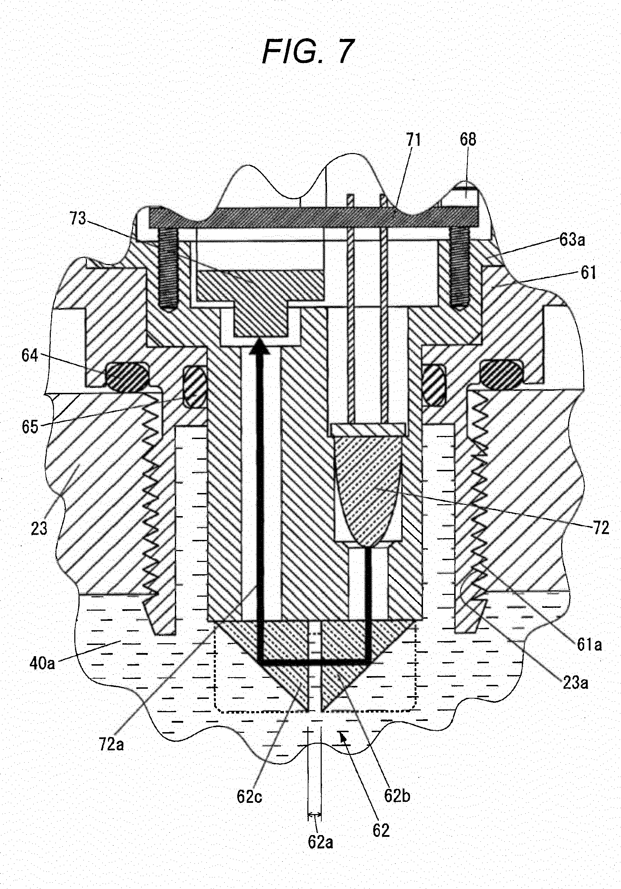

Fig. 7 is a diagram showing an optical path 72a from the white LED 72 to the RGB sensor 73.

As shown in Fig. 7, the oil gap 62a of the gap forming member 62 is disposed on the optical path 72a from the white LED 72 to the RGB sensor 73.

The holder 63a surrounds at least a part of the optical path 72a from the white LED 72 to the RGB sensor 73. The surface of the holder 63a is treated to a treatment for preventing light reflection such as a black alumite treatment for matting.

Each of the right-angle prisms 62b, 62c transmits light emitted from the white LED 72. Each of the light incident surfaces and the light emission surfaces of the right-angle prisms 62b, 62c with respect to light emitted from the white LED 72 is optically polished.

The optical path 72a is bent by 90 degrees by the light reflection surface of the right-angle prism 62b and also bent by 90 degrees by the light reflection surface of the right-angle prism 62c. That is, the optical path 72a is bent by 180 degrees by the gap forming member 62. Each of the light reflection surfaces of the right-angle prisms 62b, 62c with respect to light emitted from the white LED 72 is optically polished and provided with an aluminum deposition film. Further, an SiO2 film is formed on the aluminum deposition film in order to protect the aluminum deposition film which is low in a degree of hardness and an adhesive force.

A distance between the light emission surface of the right-angle prism 62b with respect to light emitted from the white LED 72 and the light incident surface of the right-angle prism 62c with respect to the light emitted from the white LED 72 corresponds to the length of the oil gap 62a. When the length of the oil gap 62a is too short, since the contaminant within the lubricant 40a unlikely flows through the gap 62a suitably, the detection accuracy of the colors of the contaminant within the lubricant 40a degrades. On the other hand, when the length of the oil gap 62a is too long, the light emitted from the white LED 72 is excessively absorbed by the contaminant within the lubricant 40a within the oil gap 62a and hence unlikely reaches the RGB sensor 73. Thus, the detection accuracy of the colors of the contaminant within the lubricant 40a also degrades. Accordingly, preferably, the length of the oil gap 62a is set suitably so that the detection accuracy of the colors of the contaminant within the lubricant 40a becomes high. The length of the oil gap 62a is 1 mm, for example.

The RGB sensor 73 detects colors, among the white light emitted by the white LED 72, with respect to the light having wavelengths not absorbed by the contaminant within the lubricant 40a at the oil gap 62a. Thus, the lubricant deterioration sensor 60 can immediately detect the colors of the contaminant within the lubricant 40a of the reducer 40. The reducer damage-state notification apparatus 100 can immediately specify kinds and amounts of contaminant within the lubricant 40a of the reducer 40 based on the colors detected by the RGB sensor 73. That is, the lubricant deterioration sensor 60 enables to detect deterioration degree of the lubricant 40a by detecting the colors of the contaminant within the lubricant 40a.

The deterioration degree of the lubricant 40a can be determined according to a color difference ΔE of the colors detected by the RGB sensor 73 with respect to black as a particular color. The color difference ΔE of the colors detected by the RGB sensor 73 with respect to black can be calculated by an expression shown by the following Math. 1 by using the respective values of the colors RGB detected by the RGB sensor 73.

[Math. 1]

$$\Delta E = \sqrt{R^2 + G^2 + B^2}$$

Generally, in the industrial robot, accuracy of the track of the arm etc. largely varies depending on the performance of the reducer used at the articular portion. Thus, it is important to suitably exchange the reducer for the industrial robot for new one when the performance of the reducer degrades. However, in the case of exchanging the reducer for the industrial robot, it is required to stop the industrial robot provided with this reducer and a production line mounting the industrial robot. Thus, in order to grasp the exchange time of the reducer for the industrial robot, it is very important to suitably predict a failure of the reducer for the industrial robot. In this respect, each of the lubricant deterioration sensors of the industrial robot 20 enables the reducer damage-state notification apparatus 100 to immediately specify the kinds and amounts of the contaminant within the lubricant 40a of the reducer 40 based on the colors detected by the RGB sensor 73. Thus, the machine system 10 with reducer damage-state notification function can immediately predict a failure of the reducer for the industrial robot.

To the lubricant 40a, there is sometimes added various kinds of additive such as friction reducing agent like organic molybdenum (Mo) such as molybdenum dithiocarbamate (MoDTC) or molybdenum dithiophosphate (MoDTP) for reducing the friction of a friction surface, extreme-pressure additive such as SP-based additive for improving extreme-pressure lubricity representing the performance for suppressing the sticking of the friction surface, and dispersing agent such as calcium sulfonate for suppressing the generation and adhesion of sludge. These additives are separated from the lubricant 40a in accordance with the deterioration of the lubricant 40a in such a manner that the additive adheres to, binds with or settles on the metal surface of the industrial robot 20 and the reducer. Each of the lubricant deterioration sensors enables to specify, based on the detected colors, not only an amount of ion powder within the lubricant 40a but also deterioration degree of the base oil and increase of the contaminant such as sludge due to the reduction of various types of additive added to the lubricant 40a. Thus, the machine system 10 with reducer damage-state notification function can improve the prediction accuracy of a failure as compared with a technique where a failure of the reducer is predicted only based on a density of iron powder.

Next, the assembling method of the lubricant deterioration sensor 60 will be explained.

First, each of the two right-angle prisms 62b, 62c and the white LED 72 is fixed to the holder 63a by the adhesive.

Then, the circuit board 71 mounting the RGB sensor 73 thereon is fixed to the holder 63a by means of the screws 68, and the white LED 72 is fixed to the circuit board 71 by means of soldering. Further, various kinds of electronic components such as the temperature sensor 76, the connector 79 are assembled, thereby supporting the electronic component group 70 by the holder 63a.

Next, the holder cap 63c is fixed to the holder 63a by means of the bolts 63b with hexagon holes.

Lastly, the holder 63a is fixed, by means of the bolts 67 with hexagon holes, to the housing 61 attached with the O ring 64, the O ring 65 and the O ring 66.

Next, the explanation will be made as to a method of mounting the lubricant deterioration sensor 60 to the arm 23.

First, the tool contact portion 61b of the housing 61 is grasped by a tool and the screw portion 61a of the housing 61 is threaded into the screw hole 23a of the arm 23, thereby fixing the lubricant deterioration sensor 60 to the arm 23.

Then, the connector 80 of the external device of the lubricant deterioration sensor 60 is connected to the connector 79.

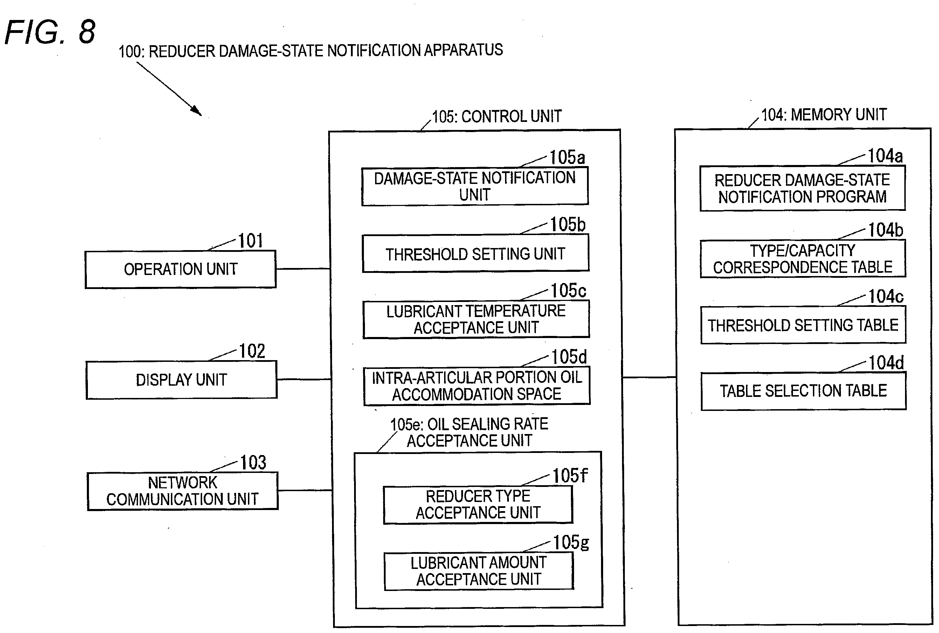

Fig. 8 is a block diagram showing the reducer damage-state notification apparatus 100.

The reducer damage-state notification apparatus 100 shown in Fig. 8 is a PC (Personal Computer). The reducer damage-state notification apparatus 100 includes an operation unit 101 which is an input device such as a mouse or a keyboard for inputting various kinds of operations by a user; a display unit 102 which is a display device such as an LCD (Liquid Crystal Display) for displaying various kinds of information; a network communication unit 103 which is a network communication device for performing communication via a network 11 (see Fig. 1); a memory unit 104 which is a memory device such as an HDD (Hard Disk Drive) for storing various kinds of data; and a control unit 105 for controlling the entirety of the reducer damage-state notification apparatus 100.

The memory unit 104 stores a reducer damage-state notification program 104a for notifying the damage state of the reducer for the industrial robot 20.

The reducer damage-state notification program 104a may be mounted into the reducer damage-state notification apparatus 100 at the manufacturing stage of the reducer damage-state notification apparatus 100. Alternatively, this program may be additionally mounted into the damage-state notification apparatus 100 from a recording medium such as a USB (Universal Serial Bus) memory, a CD (Compact Disc) or a DVD (Digital Versatile Disc). Further, alternatively, this program may be additionally mounted into the damage-state notification apparatus 100 from the network 11.

Further, the reducer damage-state notification program 104a may be executed by being downloaded from a server etc., or by reading a program recorded in a predetermined recording medium (an optical disc such as a CD or a DVD, a magnetic disc, or a semiconductor memory, for example).

A computer for executing the reducer damage-state notification program 104a may be a single or plural. That is, the program may be processed concentrically or distributedly.

The memory unit 104 stores a type/capacity correspondence table 104b which is a table representing capacities of the intra-reducer oil-accommodation space 30a respectively corresponding to the types of the reducer. In other words, the memory unit 104 constitutes a type/capacity correspondence memory unit of the invention.

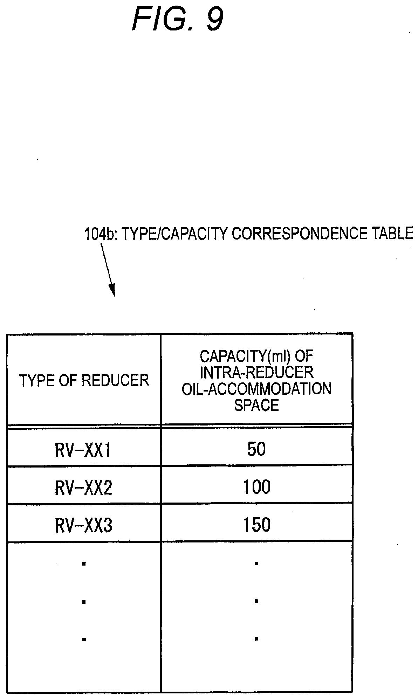

Fig. 9 is a diagram showing an example of the type/capacity correspondence table 104b.

As shown in Fig. 9, in the type/capacity correspondence table 104b, the capacities of the intra-reducer oil-accommodation space 30a are associated with the types of the reducer, respectively. According to the type/capacity correspondence table 104b shown in Fig. 9, the capacity of the intra-reducer oil-accommodation space 30a of the reducer of type "RV-XX2" is 100 ml.

As shown in Fig. 8, the memory unit 104 stores a plurality of threshold setting tables 104c each of which is a table for setting thresholds for notifying the damage state of the reducer 40. The plurality of threshold setting tables 104c stored in the memory unit 104 differ in the types of the corresponding reducers and the capacities of the intra-articular portion oil-accommodation spaces and are allocated with IDs, respectively.

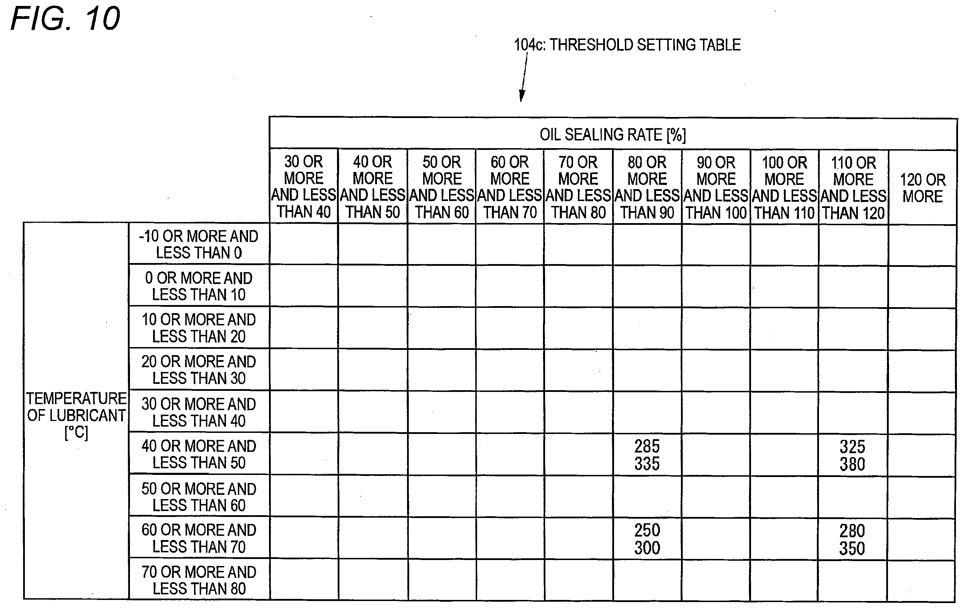

Fig. 10 is a diagram showing an example of the threshold setting table 104c. Although there are many blanks in the threshold setting table 104c shown in Fig. 10, concrete thresholds are filled therein actually.

As shown in Fig. 10, the threshold setting table 104c shows thresholds which correspond to temperatures of the lubricant 40a and oil sealing rates, respectively. The oil sealing rate represents a ratio of the amount of the lubricant 40a with respect to the capacity of the intra-reducer oil-accommodation space 30a. In the threshold setting table 104c shown in Fig. 10, numerical values are displayed in two stages in the column of the threshold. The numerical value at the upper stage represents a check/repair threshold which is a threshold for promoting checking or repairing to a user since the reducer is damaged with extremely high probability. The numerical value at the lower stage represents a warning threshold which is a threshold for warning that the reducer may be damaged. According to the threshold setting table 104c shown in Fig. 10, when the temperature of the lubricant is in a range between 40°C or more and less than 50°C and the oil sealing rate is in a range between 80% or more and less than 90%, for example, the check/repair threshold and the warning threshold are 285 and 335, respectively. In each of the case where the temperature of the lubricant 40a is less than -10°C and the case where the temperature of the lubricant 40a is 80°C or more, since these cases are out of the range of the specification of the lubricant deterioration sensor according to this embodiment, thresholds are not set in the threshold setting table 104c shown in Fig. 10. Further, in the case where the oil sealing rate is less than 30%, since this case is out of the range of the specification of the lubricant deterioration sensor according to this embodiment, thresholds are not set in the threshold setting table 104c shown in Fig. 10.

As shown in Fig. 8, the memory unit 104 stores a table selection table 104d which is a table for selecting suitable one from the plurality of threshold setting tables 104c.

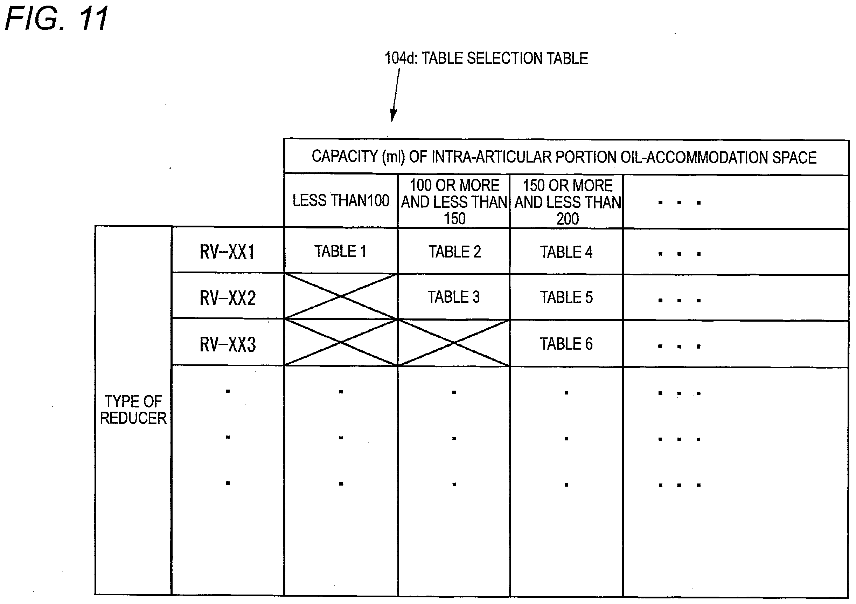

Fig. 11 is a diagram showing an example of the table selection table 104d.

As shown in Fig. 11, in the table selection table 104d, the IDs of the threshold setting tables 104c are shown in correspondence to the types of the reducer and the capacities of the intra-articular portion oil-accommodation space, respectively. According to the table selection table 104d shown in Fig. 11, when the type of the reducer is "RV-XX2" and the capacity of the intra-articular portion oil-accommodation space 30 is in a range between 100 ml or more and less than 150 ml, for example, the threshold setting table allocated with the ID "Table 3" is selected from the plurality of threshold setting tables 104c.

The control unit 105 shown in Fig. 8 includes a CPU (Central Processing Unit), an ROM (Read Only Memory) storing programs and various kinds of data in advance, and an RAM (Random Access Memory) used as the working area of the CPU, for example. The CPU executes the programs stored in the ROM or the memory unit 104.

The control unit 105 executes the reducer damage-state notification program 104a stored in the memory unit 104 to thereby function as a damage-state notification unit 105a operating as a damage-state notification means for notifying the damage state of the reducer 40;

as a threshold setting unit 105b operating as a threshold setting means for setting the threshold for performing the notification by the damage-state notification unit 105a; as a lubricant temperature acceptance unit 105c operating as a lubricant temperature acceptance means for accepting the input of the temperature of the lubricant 40a; as an intra-articular portion oil-accommodation space acceptance unit 105d operating as an oil-accommodation space acceptance means for accepting the input of the capacity of the intra-articular portion oil-accommodation space 30; and as an oil sealing rate acceptance unit 105e operating as an oil sealing rate acceptance means for accepting the input of the oil sealing rate representing the ratio of the amount of the lubricant 40a with respect to the capacity of the intra-reducer oil-accommodation space 30a. The oil sealing rate acceptance unit 105e includes a reducer type acceptance unit 105f operating as a reducer type acceptance means for accepting the input of the type of the reducer 40, and a lubricant amount acceptance unit 105g operating as a lubricant amount acceptance means for accepting the input of the amount of the lubricant 40a.

Next, an example of the method of preparing the threshold setting table 104c will be explained.

In a device for the experimentation prepared so as to have the substantially same configuration as the articular portion 32, under a condition that the maximum output rotation speed was 15 rpm, the maximum load torque was 2.5 times as large as the rated torque of the reducer 40 and a load moment was the rated moment of the reducer 40, this experimentation was performed in the following manner. That is, a reciprocal rotation movement that the supporting body 42 was rotated by 45 degrees in a reverse direction with respect to the case 41 after rotating the supporting body by 45 degrees in a forward direction with respect to the case was repeatedly continued. In this experimentation, new oil with a low deterioration degree was used as the lubricant 40a. Then, measurement is made as to the chronological change of the color difference ΔE of the colors detected by the RGB sensor 73 of the lubricant deterioration sensor 60 with respect to black.

Fig. 12(a) is a graph showing an example of the experimentation result of the chorological change of the color difference ΔE in a case where the temperature of the lubricant 40a is 40°C and the oil sealing rate is 80%. Fig. 12(b) is a graph showing an example of the experimentation result of the chorological change of the color difference ΔE in a case where the temperature of the lubricant 40a is 60°C and the oil sealing rate is 80%. Fig. 12(c) is a graph showing an example of the experimentation result of the chorological change of the color difference ΔE in a case where the temperature of the lubricant 40a is 60°C and the oil sealing rate is 110%.

In Figs. 12(a) to 12(c), a rated conversion time was obtained so that a time period during which the device for the experimentation was actually driven was converted into a time period of a case that the output rotation speed is 15 rpm and the load torque is the rated torque of the reducer 40, based on the output rotation speed and the load torque of the reducer 40 in a case where the device for the experimentation was actually driven. The life time of the reducer 40 is defined as 6,000 hours in a case where the reducer 40 is continuously driven under a condition that the output rotation speed is 15 rpm and the load torque is the rated torque of the reducer 40.

As shown in Figs. 12(a) and 12(b), even if the oil sealing rate is same, when the temperature of the lubricant 40a differs, the color difference ΔE at the time point where the rated conversion time reaches the life time of 6,000 hours (hereinafter called "color difference at rated life-time ending") differs. As shown in Figs. 12(b) and 12(c), even if the temperature of the lubricant 40a is same, when the oil sealing rate differs, the color difference at rated life-time ending differs. Thus, it is preferable to determine the thresholds for notifying the damage-state of the reducer 40 in correspondence with the temperature of the lubricant 40a and the oil sealing rate.

In the case of performing plural experimentations, even if the temperature of the lubricant 40a and the oil sealing rate is same in each of the experimentations, the color difference at rated life-time ending varies therebetween. Therefore, of the color differences at rated life-time ending obtained in the plural experimentations, the minimum value thereof is set as the check/repair threshold and the maximum value thereof is set as the warning threshold.

For example, in the threshold setting table 104c shown in Fig. 10, the check/repair threshold 285, which was a value in the case where the temperature of the lubricant is in the range between 40°C or more and less than 50°C and the oil sealing rate is in the range between 80% or more and less than 90%, was the minimum numerical value of the color differences at rated life-time ending obtained in the plural experimentations. In this respect, the plural experimentations were performed under the condition that the temperature of the lubricant is 40°C and the oil sealing rate is 80%. Similarly, in the threshold setting table 104c shown in Fig. 10, the warning threshold 335, which was a value in the case where the temperature of the lubricant is in the range between 40°C or more and less than 50°C and the oil sealing rate is in the range between 80% or more and less than 90%, was the maximum numerical value of the color differences at rated life-time ending obtained in the plural experimentations. In this respect, the plural experimentations were performed under the condition that the temperature of the lubricant is 40°C and the oil sealing rate is 80%.

The threshold setting table 104c is prepared in the aforesaid manner. The plurality of threshold setting tables 104c are prepared by performing the experimentations under conditions where the types of the reducer and the capacities of the intra-articular portion oil-accommodation space differ from each other.

Next, the operation of the machine system 10 with reducer damage-state notification function will be explained.

First, the operation of the industrial robot 20 will be explained.

Although the explanation is made hereinafter as to the articular portion 32, the operation of each of the articular portions 31, 33 to 36 is substantially same.

When the output shaft of the motor 50 of the articular portion 32 rotates, the rotation speed of the motor 50 is reduced by the reducer 40, whereby the arm 23 fixed to the supporting body 42 of the recorder 40 is moved by the rotation force of the motor with respect to the arm 22 fixed to the case 41 of the reducer 40.

The lubricant deterioration sensor 60 of the articular portion 32 emits white light from the white LED 72 in response to electric power supplied from the external device via the connector 79. Then, the lubricant deterioration sensor 60 outputs light amounts of respective colors RGB of the light received by the RGB sensor 73 as an electric signal to the external device via the connector 79. Further, the lubricant deterioration sensor 60 also outputs temperature detected by the temperature sensor 76 as an electric signal to the external device via the connector 79.

Next, the operation of the reducer damage-state notification apparatus 100 will be explained.

Although the explanation is made hereinafter as to the lubricant deterioration sensor 60 of the articular portion 32, the operation of each of the lubricant deterioration sensors of the articular portions 31, 33 to 36 is substantially same.

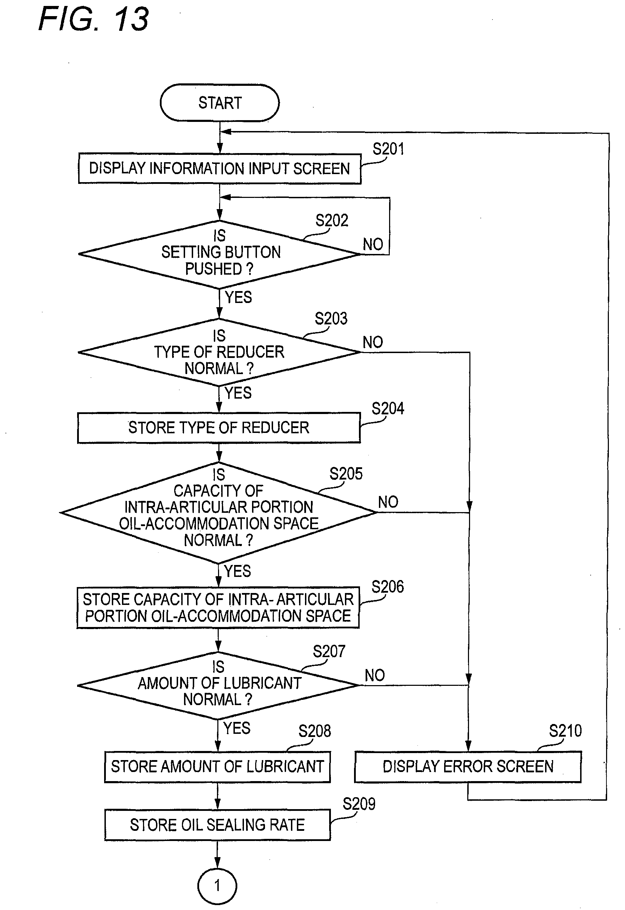

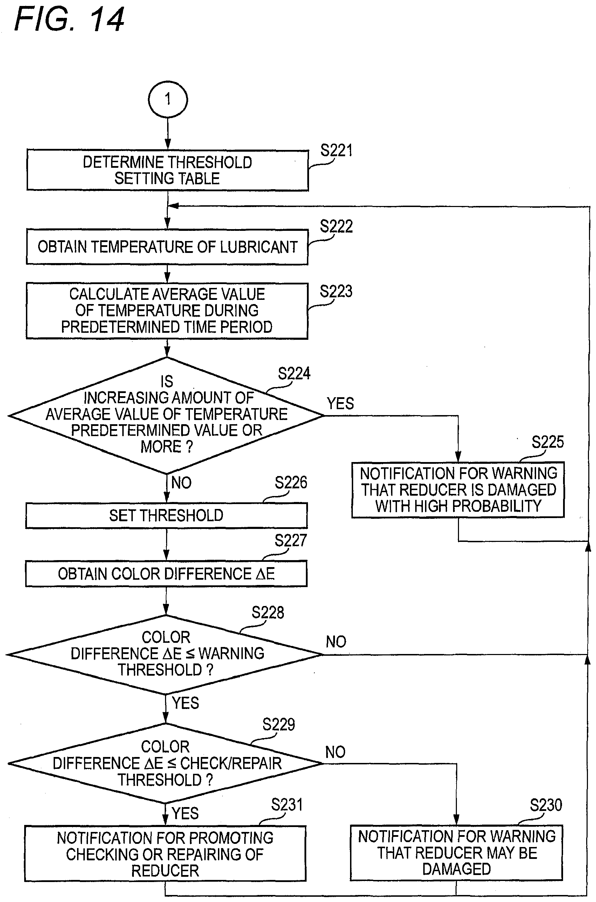

Fig. 13 is a flowchart showing the operation of the reducer damage-state notification apparatus 100. Fig.14 is a flowchart showing the succeeding operation of Fig. 13.

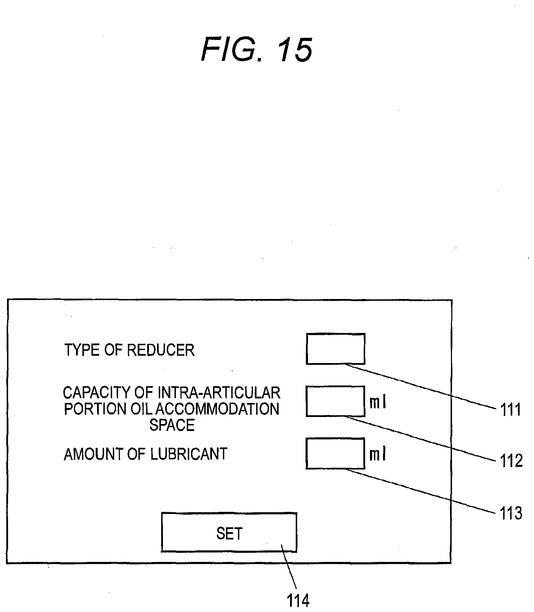

As shown in Figs. 13 and 14, the control unit 105 of the reducer damage-state notification apparatus 100 displays, on the display unit 102, an information input screen shown in Fig. 15 which is a screen for inputting various kinds of information (S201).

Fig. 15 is a diagram showing an example of the information input screen displayed on the display unit 102.

The information input screen shown in Fig. 15 includes a text box 111 for inputting the type of the reducer 40, a text box 112 for inputting the capacity of the intra-articular portion oil-accommodation space 30, a text box 113 for inputting an amount of the lubricant 40a sealed into the intra-articular portion oil-accommodation space 30, and a setting button 114 for setting the information inputted into the text boxes 111 to 113. A user can input suitable information into the text boxes 111 to 113 via the operation unit 101 and push the setting button 114.

As shown in Figs. 13 and 14, the control unit 105 determines whether or not the setting button 114 is pushed, until it is determined that the setting button 114 is pushed (S202).

When it is determined in S202 that the setting button 114 is pushed, the reducer type acceptance unit 105f of the control unit 105 determines whether or not the type of the reducer 40 inputted into the text box 111 is normal (S203). In this respect, the reducer type acceptance unit 105f determines that the type of the reducer 40 inputted into the text box 111 is normal only when the type of the reducer 40 contained in the type/capacity correspondence table 104b of the memory unit 104 is inputted into the text box 111.

When the reducer type acceptance unit 105f determines in S203 that the type of the reducer 40 inputted into the text box 111 is normal, this acceptance unit stores the type of the reducer 40 inputted into the text box 111 (S204). That is, the reducer type acceptance unit 105f accepts the input of the type of the reducer 40.

Next, the intra-articular portion oil-accommodation space acceptance unit 105d of the control unit 105 determines whether or not the capacity of the intra-articular portion oil-accommodation space 30 inputted into the text box 112 is normal (S205). In this respect, the intra-articular portion oil-accommodation space acceptance unit 105d determines that the capacity of the intra-articular portion oil-accommodation space 30 inputted into the text box 112 is normal, only when the capacity inputted into the text box 112 is equal to or larger than the capacity of the intra-reducer oil-accommodation space 30a associated with the type of the reducer 40 stored in S204 on the type/capacity correspondence table 104b of the memory unit 104.

When the intra-articular portion oil-accommodation space acceptance unit 105d determines in S205 that the capacity of the intra-articular portion oil-accommodation space 30 inputted into the text box 112 is normal, this acceptance unit stores the capacity of the intra-articular portion oil-accommodation space 30 inputted into the text box 112 (S206). That is, the intra-articular portion oil-accommodation space acceptance unit 105d accepts the input of the capacity of the intra- articular portion oil-accommodation space 30.

Next, the lubricant amount acceptance unit 105g of the control unit 105 determines whether or not the amount of the lubricant 40a inputted into the text box 113 is normal (S207). In this respect, the lubricant amount acceptance unit 105g determines that the amount of the lubricant 40a inputted into the text box 113 is normal, only in the case that the amount of the lubricant 40a inputted into the text box 113 is 30% or more of the capacity of the intra-reducer oil-accommodation space 30a associated with the type of the reducer 40 stored in S204 on the type/capacity correspondence table 104b of the memory unit 104 and that the amount of the lubricant 40a inputted into the text box 113 is less than the capacity of the intra-articular portion oil-accommodation space 30 stored in S206.

When the lubricant amount acceptance unit 105g determines in S207 that the amount of the lubricant 40a inputted into the text box 113 is normal, this acceptance unit stores the amount of the lubricant 40a inputted into the text box 113 (S208). That is, the lubricant amount acceptance unit 105g accepts the input of the amount of the lubricant 40a.

Next, the oil sealing rate acceptance unit 105e of the control unit 105 calculates the oil sealing rate based on the amount of the lubricant 40a stored in S208 and the capacity of the intra-reducer oil-accommodation space 30a associated with the type of the reducer 40 stored in S204 on the type/capacity correspondence table 104b of the memory unit 104, and stores the calculated oil sealing rate (S209). That is, the oil sealing rate acceptance unit 105e accepts the input of the oil sealing rate.

The control unit 105 displays an error screen on the display unit 102 when it is determined in S203 that the type of the reducer 40 inputted into the text box 111 is not normal, or when it is determined in S205 that the capacity of the intra-articular portion oil-accommodation space 30 inputted into the text box 112 is not normal, or when it is determined in S207 that the amount of the lubricant 40a inputted into the text box 113 is not normal (S210), and returns again to the processing S201.

After executing the processing S209, the control unit 105 obtains the ID of the threshold setting table 104c which is associated with the type of the reducer 40 stored in S204 and the capacity of the intra-articular portion oil-accommodation space 30 stored in S206 on the table selection table 104d of the memory unit 104. Then, the control unit determines the threshold setting table 104c allocated with the ID thus obtained as the threshold setting table to be used in the succeeding processing (S221).

Next, the lubricant temperature acceptance unit 105c of the control unit 105 obtains the detection result of the temperature sensor 76 of the lubricant deterioration sensor 60 via the network communication unit 103 (S222). That is, the lubricant temperature acceptance unit 105c accepts the input of the temperature of the lubricant 40a.

Next, the control unit 105 calculates an average value of the temperature of the lubricant 40a obtained by plural times of processing S222 during a predetermined time period such as 10 minutes (S223). That is, the control unit 105 calculates an average value of the temperature of the lubricant 40a based on not only the temperature of the lubricant 40a obtained in the just before processing S222 but also the temperature of the lubricant 40a obtained in at least one most recent processing S222 which is executed before the just before processing S222.

Next, the damage-state notification unit 105a of the control unit 105 determines whether or not an increasing amount of the average value of the temperature of the lubricant 40a calculated in S223 is a predetermined value or more, for example, 20°C or more (S224). In other words, the damage-state notification unit 105a of the control unit 105 determines whether or not the calculated average value of the temperature of the lubricant 40a during the current predetermined time period is increased by the predetermined value or more with respect to the calculated average value of the temperature of the lubricant 40a during the just previous predetermined time period

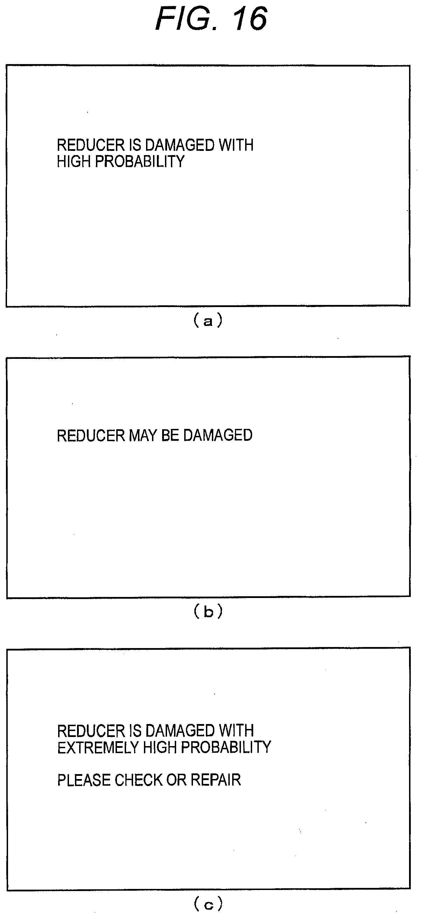

When it is determined in S224 that the increasing amount of the average value of the temperature of the lubricant 40a calculated in S223 is the predetermined value or more, the damage-state notification unit 105a displays on the display unit 102 a notification warning that the reducer 40 is damaged with high probability as shown in Fig. 16(a), as the notification representing the damage-state of the reducer 40 (S225). Then, the processing returns again to S222.

On the other hand, when it is determined in S224 that the increasing amount of the average value of the temperature of the lubricant 40a calculated in S223 is less than the predetermined value, the threshold setting unit 105b of the control unit 105 sets the warning threshold and the check/repair threshold as the thresholds to be used in the succeeding processing (S226). In this respect, these warning threshold and check/repair threshold thus set are associated in the threshold setting table 104c which is determined in S221 with respect to the oil sealing rate stored in S209 and the average value of the temperature of the lubricant 40a calculated in S223.

Next, the damage-state notification unit 105a obtains the detection result of the RGB sensor 73 of the lubricant deterioration sensor 60, that is, the colors of the lubricant 40a via the network communication unit 103, and calculates and obtains the color difference ΔE of the obtained colors with respect to black based on the aforesaid Math. 1 (S227).

Next, the damage-state notification unit 105a determines whether or not the color difference ΔE obtained in S227 is equal to or less than the warning threshold set in S226 (S228).

When the damage-state notification unit 105a determines in S228 that the color difference ΔE obtained in S227 is not equal to or less than the warning threshold set in S226, the processing returns again to S222.

On the other hand, when the damage-state notification unit 105a determines in S228 that the color difference ΔE obtained in S227 is equal to or less than the warning threshold set in S226, the damage-state notification unit determines whether or not the color difference ΔE obtained in S227 is equal to or less than the check/repair threshold set in S226 (S229).

When the damage-state notification unit 105a determines in S229 that the color difference ΔE obtained in S227 is not equal to or less than the check/repair threshold set in S226, as shown in Fig. 16(b), the damage-state notification unit displays on the display unit 102 a notification warning that the reducer 40 may be damaged, as the notification representing the damage state of the reducer 40 (S230). That is, when the color difference ΔE between the colors detected by the RGB sensor 73 and black reaches the warning threshold, the damage-state notification unit 105a notifies the damage state of the reducer 40 corresponding to the warning threshold. The control unit 105 returns the processing again to S222 after performing the processing S230.

On the other hand, when the damage-state notification unit 105a determines in S229 that the color difference ∆E obtained in S227 is equal to or less than the check/repair threshold set in S226, as shown in Fig. 16(c), the damage-state notification unit displays on the display unit 102 a notification for promoting checking or repairing of the reducer 40 to a user, as the notification representing the damage state of the reducer 40 (S231). That is, when the color difference ∆E between the colors detected by the RGB sensor 73 and black reaches the check/repair threshold, the damage-state notification unit 105a notifies the damage state of the reducer 40 corresponding to the check/repair threshold. The control unit 105 returns the processing again to S222 after performing the processing S231.

As explained above, the reducer damage-state notification apparatus 100 sets the warning threshold and the check/repair threshold (S226) in accordance with the temperature of the lubricant 40a (S222) and the oil sealing rate (S209). Thus, even in the case that the temperature of the lubricant 40a and the oil sealing rate as the working condition of the reducer 40 are different, the damage state of the reducer 40 can be notified accurately. Even in the case that the reducer damage-state notification apparatus 100 is configured not to set the warning threshold and the check/repair threshold in accordance with either one of the temperature of the lubricant 40a and the oil sealing rate, the damage state of the reducer 40 can be notified accurately although the accuracy is degraded as compared with the configuration that the warning threshold and the check/repair threshold are set in accordance with the temperature of the lubricant 40a and the oil sealing rate.

The reducer damage-state notification apparatus 100 sets the warning threshold and the check/repair threshold (S226) in accordance with the actual temperature of the lubricant 40a detected by the temperature sensor 76 (S222). Thus, the damage state of the reducer 40 can be notified accurately in accordance with the actual temperature of the lubricant 40a. The reducer damage-state notification apparatus 100 may be configured to set the warning threshold and the check/repair threshold in accordance with the temperature of the lubricant 40a other than the actual temperature of the lubricant 40a detected by the temperature sensor 76. For example, the reducer damage-state notification apparatus 100 may set the warning threshold and the check/repair threshold in accordance with the temperature of the lubricant 40a inputted by a user via the operation unit 101.

The reducer damage-state notification apparatus 100 sets the warning threshold and the check/repair threshold (S226) in accordance with the average value of the actual temperature of the lubricant 40a during the predetermined time period (S222 and S223). Thus, even in a case that the temperature of the lubricant 40a rapidly changes temporarily, erroneous notification of the damage state of the reducer 40 can be prevented. As the temperature of the lubricant 40a used in S226, instead of the average value of the actual temperature of the lubricant 40a during the predetermined time period, the reducer damage-state notification apparatus 100 may employ only the actual temperature of the lubricant 40a obtained in the just before processing S222.

In such a case just after the exchange of the lubricant 40a for new one, the lubricant 40a may not deteriorate immediately even when the reducer 40 approaches actually to a damaged state or has been already damaged. When the lubricant 40a is not deteriorated yet, the color difference ∆E between the colors detected by the RGB sensor 73 and black does not reach the warning threshold nor the check/repair threshold. However, when the reducer 40 approaches actually to a damaged state or has been already damaged, since the reducer 40 intensely generates heat, the temperature of the lubricant 40a increases rapidly. The reducer damage-state notification apparatus 100 notifies the damage state of the reducer 40 (S225) in the case where the increasing amount of the average value of the actual temperature of the lubricant 40a during the predetermined time period (S222 and S223) is the predetermined value or more (Yes in S224). Thus, when the reducer 40 approaches actually to a damaged state or has been already damaged, even if the color difference ∆E between the colors detected by the RGB sensor 73 and black does not reach the warning threshold nor the check/repair threshold, the damage state of the reducer 40 can be notified suitably. The reducer damage-state notification apparatus 100 may be configured to execute the processing S226 immediately after the processing S223 without executing the processing S224 and S225.

Although the temperature sensor 76 is configured to detect the temperature of the lubricant 40a via the holder 63a, the temperature sensor may be configured to directly detect the temperature of the lubricant 40a without interposing other member such as the holder 63a.

Since the temperature sensor 76 is contained within the lubricant deterioration sensor 60, the machine system 10 with reducer damage-state notification function can be configured easily as compared with a configuration where the temperature sensor 76 is provided separately from the lubricant deterioration sensor 60. Incidentally, in the machine system 10 with reducer damage-state notification function, the temperature sensor 76 may be provided separately from the lubricant deterioration sensor 60.

The relation between the damage state of the reducer 40 and the deterioration state of the lubricant 40a changes depending on the rated torque of the reducer 40 as well as the oil sealing rate. The rated torque of the reducer 40 is determined according to the type of the reducer 40. The threshold setting unit 105b sets the warning threshold and the check/repair threshold further according to the type of the reducer 40 which input is accepted by the reducer type acceptance unit 105f (S221). Thus, even in the case that the type of the reducer 40 differs, the damage state of the reducer 40 can be notified accurately. Further, the oil sealing rate acceptance unit 105e calculates the oil sealing rate (S209) based on the capacity of the intra-reducer oil-accommodation space 30a, which is stored in the memory unit 104 in association with the type of the reducer 40 (S204) which input is accepted by the reducer type acceptance unit 105f, and the amount of the lubricant 40a (S208) which input is accepted by the lubricant amount acceptance unit 105g, whereby the input of the oil sealing rate can be indirectly accepted. Thus, the reducer damage-state notification apparatus 100 can set the warning threshold and the check/repair threshold according to the oil sealing rate and the type of the reducer 40 by merely inputting the amount of the lubricant 40a and the type of the reducer 40.

When the capacity of the intra-reducer oil-accommodation space 30a is arranged to be inputted directly from the operation unit 101, the oil sealing rate acceptance unit 105e may calculate the oil sealing rate based on the capacity of the intra-reducer oil-accommodation space 30a which input is directly accepted and the amount of the lubricant 40a which input is accepted by the lubricant amount acceptance unit 105g, whereby the input of the oil sealing rate may be indirectly accepted. The oil sealing rate acceptance unit 105e may be configured to directly input the oil sealing rate itself from the operation unit 101.

The reducer damage-state notification apparatus 100 may be configured not to set the warning threshold and the check/repair threshold according to the type of the reducer 40.

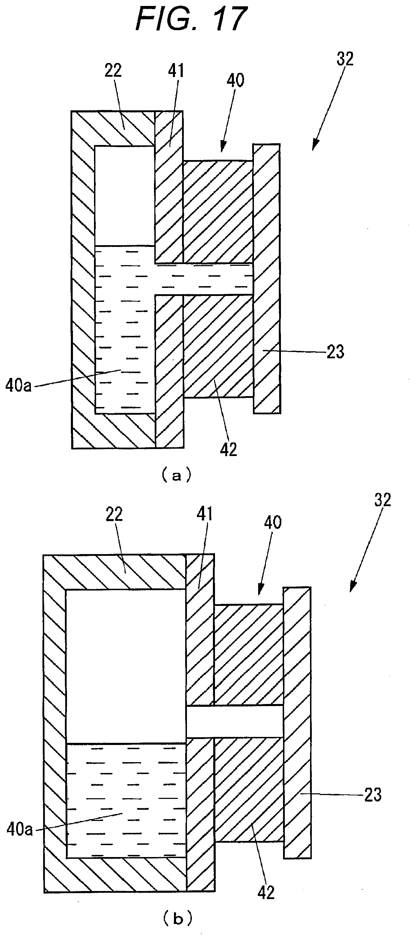

Fig. 17(a) is a schematic diagram of the articular portion 32 in a case that the capacity of the intra-articular portion oil-accommodation space 30, the capacity of the intra-reducer oil-accommodation space 30a, and the amount of the lubricant 40a are 200 ml, 150 ml and 120 ml, respectively. Fig. 17(b) is a schematic diagram of the articular portion 32 in a case that the capacity of the intra-articular portion oil-accommodation space 30, the capacity of the intra-reducer oil-accommodation space 30a, and the amount of the lubricant 40a are 300 ml, 150 ml and 120 ml, respectively.

Concerning the oil sealing rate of the articular portion 32 shown in Fig. 17(a) and the oil sealing rate of the articular portion 32 shown in Fig. 17(b), since the oil sealing rate is a ratio of the amount 120 ml of the lubricant 40a with respect to the capacity 150 ml of the intra-reducer oil-accommodation space 30a in each of these cases, the oil sealing rate is 80% in each of these case. However, between the articular portion 32 shown in Fig. 17(a) and the articular portion 32 shown in Fig. 17(b), the oil sealing rate is same but the capacity of the intra-articular portion oil-accommodation space 30 is different.

Thus, the amount of the lubricant 40a actually present within the reducer 40 differs between these cases. Thus, the relation between the damage state of the reducer 40 and the deterioration state of the lubricant 40a differs between the articular portion 32 shown in Fig. 17(a) and the articular portion 32 shown in Fig. 17(b). However, in the reducer damage-state notification apparatus 100, the warning threshold and the check/repair threshold are set in accordance with the capacity of the intra-articular portion oil-accommodation space 30 (S221). Therefore, even when the capacity of the intra-articular portion oil-accommodation space 30 is different as the working condition of the reducer 40, the damage state of the reducer 40 can be notified accurately. The reducer damage-state notification apparatus 100 may be configured not to set the warning threshold and the check/repair threshold according to the capacity of the intra-articular portion oil-accommodation space 30.

In the reducer damage-state notification apparatus 100, the two levels of the thresholds, that is, the warning threshold and the check/repair threshold corresponding to the probability of the damage of the reducer 40 are contained as the thresholds for notifying the damage state of the reducer 40. Thus, the damage state of the reducer 40 can be notified in the two levels of the probability of the damage of the reducer 40 (S230, S231). When the damage state of the reducer 40 is notified in plural levels of the probability of the damage of the reducer 40, a user can determine the necessity of exchanging the reducer 40 with sufficient time, as compared with a configuration where it is notified suddenly that the reducer 40 is damaged with extremely high probability, for example. In the reducer damage-state notification apparatus 100, the threshold for notifying the damage state of the reducer 40 may be single. Alternatively, three or more levels of the thresholds corresponding to the probability of the damage of the reducer 40 may be contained as the threshold for notifying the damage state of the reducer 40.

In this embodiment, although the reducer damage-state notification apparatus 100 is configure to execute the notification by means of the display, the notification may be executed by means of a method other than the display such as an audio output in addition to or in place of the display.

Although the reducer damage-state notification apparatus according to the invention is a PC for executing the reducer damage-state notification program in this embodiment, the apparatus may be realized by other configuration. For example, the reducer damage-state notification apparatus according to the invention may be realized by a control panel for controlling the operation of the industrial robot 20, or a computer for managing the operation of a plurality of the industrial robots 20.

Although the machine according to the invention is the industrial robot in this embodiment, a machine other than the industrial robot may be used.

Industrial Applicability

According to the invention, the reducer damage-state notification apparatus can be provided which can notify the damage state of the reducer accurately even when the working condition of the reducer differs.

Reference Signs List

    10 machine system with reducer damage-state notification function
    20 industrial robot (machine)
    30 intra-articular portion oil-accommodation space (oil accommodation space)
    30a intra-reducer oil-accommodation space
    40 reducer
    40a lubricant
    60 lubricant deterioration sensor
    62 gap forming member
    62a oil gap
    72 white LED (light emitting element)
    72a optical path
    73 RGB sensor (color light reception element)
    76 temperature sensor (lubricant temperature sensor)
    100 reducer damage-state notification apparatus
    104 memory unit (type/capacity correspondence memory unit)
    104a reducer damage-state notification program
    105a damage-state notification unit (damage-state notification means)
    105b threshold setting unit (threshold setting means)
    105c lubricant temperature acceptance unit (lubricant temperature acceptance means)
    105d intra-articular portion oil-accommodation space acceptance unit (oil-accommodation space acceptance means)
    105e oil sealing rate acceptance unit (oil sealing rate acceptance means)
    105f reducer type acceptance unit (reducer type acceptance means)
    105g lubricant amount acceptance unit (lubricant amount acceptance means)

**Claims**

1. A reducer damage-state notification apparatus for notifying a damage state of a reducer of a machine which includes the reducer (40) and a lubricant deterioration sensor (60) for detecting deterioration of lubricant of the reducer (40), wherein
the machine is formed with an oil accommodation space (30) which is a space capable of accommodating the lubricant,
the reducer (40) is formed with an intra-reducer oil-accommodation space (30a) as a part of the oil accommodation space (30),
the lubricant deterioration sensor (60) includes:

    a light emitting element (72) configured to emit light;
    a color light reception element (72) configured to detect color of received light; and
    a gap forming member (62) disposed on an optical path (72a) from the light emitting element (72) to the color light reception element (72), the gap forming member (62) forming an oil gap into which the lubricant enters and configured to transmit light therethrough,
**characterized in that**
the reducer damage-state notification apparatus comprises:

    a damage-state notification unit (105a) configured to notify the damage state of the reducer (40);
    a threshold setting unit (105b) configured to set a threshold for performing notification by the damage-state notification unit (105a); and
    an oil sealing rate acceptance unit (105e) configured to accept an input of an oil sealing rate representing a ratio of an amount of the lubricant with respect to a capacity of the intra-reducer oil-accommodation space (30a), wherein
    the damage-state notification unit (105a) is configured to notify the damage state of the reducer (40) corresponding to the threshold when a color difference between the color detected by the color light reception element (72) and a predetermined color reaches the threshold set by the threshold setting unit (105b), and
    the threshold setting unit (105b) is adapted to set the threshold according to the oil sealing rate which input is accepted by the oil sealing rate acceptance unit (105e).

2. The reducer damage-state notification apparatus according to claim 1, further comprising:

    a lubricant temperature acceptance unit (105c) configured to accept an input of temperature of the lubricant, wherein
    the threshold setting unit (105b) is configured to set the threshold according to the temperature of the lubricant which input is accepted by the lubricant temperature acceptance unit (105c).

3. The reducer damage-state notification apparatus according to claim 2, wherein
the machine includes a lubricant temperature sensor (76) for detecting the temperature of the lubricant, and

the lubricant temperature acceptance unit (105c) is configured to accept an input of temperature of the lubricant detected by the lubricant temperature sensor (76).

4. The reducer damage-state notification apparatus according to claim 3, wherein
the threshold setting unit (105b) is configured to set the threshold according to an average value of the temperature of the lubricant, during a predetermined time period, which input is accepted by the lubricant temperature acceptance unit (105c).

5. The reducer damage-state notification apparatus according to claim 3, wherein the damage-state notification unit is configured to notify the damage state of the reducer (40) when an increasing amount of an average value of the temperature of the lubricant during a predetermined time period, which input is accepted by the lubricant temperature acceptance unit (105c), is a predetermined value or more.

6. The reducer damage-state notification apparatus according to claim any of claims 1 to 5, further comprising
a type/capacity correspondence memory unit (104) configured to store a capacity of the intra-reducer oil-accommodation space (30a) corresponding to a type of the reducer (40), wherein
the oil sealing rate acceptance unit (105e) includes a reducer type acceptance unit (105f) configured to accept an input of the type of the reducer (40), and a lubricant amount acceptance unit (105g) configured to accept an input of an amount of the lubricant, the oil sealing rate acceptance unit (105e) indirectly accepts the input of the oil sealing rate by calculating the oil sealing rate based on the capacity of the intra-reducer oil-accommodation space (30a), which is stored in the type/capacity correspondence memory unit (104) in correspondence to the type of the reducer (40) which input is accepted by the reducer type acceptance unit (105f), and the amount of the lubricant which input is accepted by the lubricant amount acceptance unit (105g), and
the threshold setting unit (105b) is configured to set the threshold further according to the type of the reducer (40) which input is accepted by the reducer type acceptance unit (105f).

7. The reducer damage-state notification apparatus according to any one of claims 1 to 5, further comprising
an oil-accommodation space acceptance unit (105d) configured to accept an input of a capacity of the oil accommodation space (30), wherein
the threshold setting unit (105b) is configured to set the threshold further according to the capacity of the oil accommodation space (30) which input is accepted by the oil-accommodation space acceptance unit (105d).

8. The reducer damage-state notification apparatus according to any one of claims 1 to 7, wherein the threshold includes plural levels of thresholds corresponding to probability of the damage of the reducer (40).

9. A machine system with reducer damage-state notification function, comprising:

the reducer damage-state notification apparatus according to any one of claims 1 to 8; and
the machine to which the damage state of the reducer (40) is notified by the reducer damage-state notification apparatus.

10. A machine system with reducer damage-state notification function, comprising:

the reducer damage-state notification apparatus according to any one of claims 3 to 5; and
the machine to which the damage state of the reducer (40) is notified by the reducer damage-state notification apparatus, wherein
the lubricant temperature sensor (76) is contained in the lubricant deterioration sensor (60).

11. A medium which records a reducer damage-state notification program (104a) for making a computer function as the reducer damage-state notification apparatus according to any one of claims 1 to 8.

**Patentansprüche**

1. Untersetzungsgetriebeschadenszustand-Berichtsvorrichtung zum Berichten eines Schadenszustands eines Untersetzungsgetriebes einer Maschine, die das Untersetzungsgetriebe (40) und einen Schmiermittelalterungssensor (60) zur Erfassung einer Alterung eines Schmiermittels des Untersetzungsgetriebes (40) aufweist, wobei
die Maschine mit einem Ölaufnahmeraum (30) versehen ist, der ein Raum ist, der zur Aufnahme des Schmiermittels geeignet ist,
das Untersetzungsgetriebe (40) mit einem untersetzungsgetriebeinternen Ölaufnahmeraum (30a) als Teil des Ölaufnahmeraums (30) versehen ist,
der Schmiermittelalterungssensor (60) aufweist:

ein Licht emittierendes Element (72), das zur Aussendung von Licht ausgebildet ist;
ein Farblichtempfangselement (72), das ausgebildet ist, eine Farbe von empfangenem Licht zu erfassen; und

ein Spaltbildungselement (62), das in einem optischen Weg (72a) zwischen dem Licht emittierenden Element (72) und dem Farblichtempfangselement (72) angeordnet ist, wobei das Spaltbildungselement (62) einen Ölspalt bildet, in den das Schmiermittel eintritt und der geeignet ist, Licht durchzulassen,
**dadurch gekennzeichnet, dass**
die Untersetzungsgetriebeschadenszustand-Berichtsvorrichtung umfasst:

eine Schadenzustands-Berichtseinheit (105a), die ausgebildet ist, den Schadenszustand des Untersetzungsgetriebes (40) zu berichten;
eine Schwellenwerteinstelleinheit (105b), die ausgebildet ist, einen Schwellenwert für das Berichten durch die Schadenszustands-Berichtseinheit (105a) festzulegen; und eine Öldichtungsratenaufnahmeeinheit (105e), die ausgebildet ist, als Eingabe eine Ölabdichtungsrate zu empfangen, die ein Verhältnis einer Menge des Schmiermittels in Bezug auf eine Kapazität des untersetzungsgetriebeinternen Ölaufnahmeraums (30a) repräsentiert, wobei
die Schadenszustands-Berichtseinheit (105a) ausgebildet ist, den Schadenszustand des Untersetzungsgetriebes (40) entsprechend dem Schwellenwert zu berichten, wenn eine Farbdifferenz zwischen der von dem Farblichtaufnahmeelement (72) erfassten Farbe und einer vorbestimmten Farbe den von dem Schwellenwerteinstelleinheit (105b) festgelegten Schwellenwert erreicht, und
die Schwellenwerteinstelleinheit (105b) ausgebildet ist, den Schwellenwert entsprechend der Ölabdichtungsrate festzulegen, die als Eingabe von der Ölabdichtungsratenaufnahmeeinheit (105e) empfangen wird.

2. Untersetzungsgetriebeschadenszustands-Berichtsvorrichtung nach Anspruch 1, die ferner umfasst:

eine Schmiermitteltemperaturaufnahmeeinheit (105c), die ausgebildet ist, als Eingabe die Temperatur des Schmiermittels zu empfangen, wobei
die Schwellenwerteinstelleinheit (105b) ausgebildet ist, den Schwellenwert gemäß der Temperatur des Schmiermittels festzulegen, die als Eingabe von der Schmiermitteltemperaturaufnahmeeinheit (105c) empfangen wird.

3. Untersetzungsgetriebeschadenszustands-Berichtsvorrichtung nach Anspruch 2, wobei
die Maschine einen Schmiermitteltemperatursensor (76) zur Erfassung der Temperatur des Schmiermittels aufweist, und
die Schmiermitteltemperaturaufnahmeeinheit (105c) ausgebildet ist, als Eingabe die Temperatur des Schmiermittels, die von dem Schmiermitteltemperatursensor (76) erfasst ist, zu empfangen.

4. Untersetzungsgetriebeschadenszustands-Berichtsvorrichtung nach Anspruch 3, wobei
die Schwellenwerteinstelleinheit (105b) ausgebildet ist, den Schwellenwert gemäß einem Mittelwert der Temperatur des Schmiermittels während einer vorbestimmten Zeitdauer festzulegen, die als Eingabe von der Schmiermitteltemperaturaufnahmeeinheit (105c) empfangen wird.

5. Untersetzungsgetriebeschadenszustands-Berichtsvorrichtung nach Anspruch 3, wobei die Schadenszustands-Berichtseinheit ausgebildet ist, den Schadenszustand des Untersetzungsgetriebe (40) zu berichten, wenn ein steigender Wert eines Mittelwerts der Temperatur des Schmiermittels während einer vorbestimmten Zeitdauer, der als Eingabe von der Schmiermitteltemperaturaufnahmeeinheit (105c) empfangen wird, gleich einem vorbestimmten Wert oder größer ist.

6. Untersetzungsgetriebeschadenszustands-Berichtsvorrichtung nach einem der Ansprüche 1 bis 5, die ferner aufweist
eine Typ/Kapazitätszuordnungsspeichereinheit (104), die ausgebildet ist, eine Kapazität des untersetzungsgetriebeinternen Ölaufnahmeraums (30a) entsprechend einem Typ des Untersetzungsgetriebes (40) zu speichern, wobei
die Ölabdichtungsratenaufnahmeeinheit (105e) eine Untersetzungsgetriebetyp/Aufnahmeeinheit (105f) aufweist, die ausgebildet ist, als Eingabe den Typ des Untersetzungsgetriebes (40) zu empfangen, und eine Schmiermittelmengenaufnahmeeinheit (105g) aufweist, die ausgebildet ist, als Eingabe eine Menge des Schmiermittels zu empfangen,
die Ölabdichtungsratenaufnahmeeinheit (105e) indirekt als Eingabe die Ölabdichtungsrate empfängt durch Berechnung der Ölabdichtungsrate auf der Grundlage der Kapazität des untersetzungsgetriebeinternen Ölaufnahmeraums (30a), die in der Typ/Kapazitätszuordnungsspeichereinheit (104) zugeordnet zu dem Typ des Untersetzungsgetriebes (40) gespeichert ist, der als Eingabe von der Untersetzungsgetriebetyp/Aufnahmeeinheit (105f) empfangen wird, und auf der Grundlage der Menge des Schmiermittels, die als Eingabe von der Schmiermittelmengenaufnahmeeinheit (105g) empfangen wird, und
die Schwellenwerteinstelleinheit (105b) ausgebildet ist, den Schwellenwert ferner gemäß dem Typ des Untersetzungsgetriebes (40), der als Eingabe von der Untersetzungsgetriebetyp/Aufnahmeeinheit (105f) empfangen wird, festzulegen.

7. Untersetzungsgetriebeschadenszustands-Berichtsvorrichtung nach einem der Ansprüche 1 bis 5, die ferner aufweist
eine Ölaufnahmeraumaufnahmeeinheit (105d), die ausgebildet ist, als Eingabe eine Kapazität des Ölaufnahmeraums (30) zu empfangen, wobei die Schwellenwerteinstelleinheit (105b) ausgebildet ist, einen Schwellenwert ferner gemäß der Kapazität des Ölaufnahmeraums (30), die als Eingabe von der Ölaufnahmeraumaufnahmeeinheit (105d) empfangen wird, festzulegen.

8. Untersetzungsgetriebeschadenszustands-Berichtsvorrichtung nach einem der Ansprüche 1 bis 7, wobei der Schwellenwert mehrere Schwellenstufen entsprechend einer Wahrscheinlichkeit der Schädigung des Untersetzungsgetriebes (40) beinhaltet.

9. Maschinensystem mit einer Untersetzungsgetriebeschadenzustands-Berichtsfunktion, mit:

   einer Untersetzungsgetriebeschadenszustands-Berichtsvorrichtung nach einem der Ansprüche 1 bis 8; und
   der Maschine, der der Schadenszustand des Untersetzungsgetriebes (40) von der Untersetzungsgetriebeschadenszustands-Berichtsvorrichtung berichtet wird.

10. Maschinensystem mit einer Untersetzungsgetriebeschadenszustands-Berichtsfunktion, mit:

    der Untersetzungsgetriebeschadenszustands-Berichtsvorrichtung nach einem der Ansprüche 3 bis 5; und
    der Maschine, der der Schadenszustand des Untersetzungsgetriebes (40) von der Untersetzungsgetriebeschadenszustands-Berichtsvorrichtung berichtet wird, wobei der Schmiermitteltemperatursensor (76) in dem Schmiermittelalterungssensor (60) enthalten ist.

11. Medium, das ein Programm zum Berichten eines Untersetzungsgetriebeschadenszustand (104a) speichert, um zu veranlassen, dass ein Computer als die Untersetzungsgetriebeschadenszustands-Berichtsvorrichtung nach einem der Ansprüche 1 bis 8 fungiert.

**Revendications**

1. Dispositif de notification d'état d'endommagement de réducteur pour notifier un état d'endommagement d'un réducteur d'une machine qui comprend le réducteur (40) et un capteur de dégradation de lubrifiant (60) pour une détection d'une dégradation d'un lubrifiant du réducteur (40), dans lequel
la machine est constituée d'un compartiment d'accueil d'huile (30) qui correspond à un espace capable d'accueillir le lubrifiant,
le réducteur (40) est constitué d'un compartiment d'accueil d'huile intra-réducteur (30a) en tant qu'un élément du compartiment d'accueil d'huile (30),
le capteur de dégradation de lubrifiant (60) comprend :

   un élément d'émission de lumière (72) configuré pour émettre une lumière ;
   un élément de réception de lumière de couleur (72) configuré pour détecter une couleur d'une lumière reçue ; et
   un élément formant un vide (62) disposé sur une trajectoire optique (72a) allant de l'élément d'émission de lumière (72) à l'élément de réception de lumière de couleur (72), l'élément formant un vide (62) formant un vide d'huile dans lequel le lubrifiant pénètre et configuré pour transmettre la lumière à travers celui-ci,
   **caractérisé en ce que**
   le dispositif de notification d'état d'endommagement de réducteur comprend :

      une unité de notification d'état d'endommagement (105a) configurée pour notifier l'état d'endommagement du réducteur (40) ;
      une unité de paramétrage de seuil (105b) configurée pour définir un seuil pour procéder à une notification par l'unité de notification d'état d'endommagement (105a) ; et
      une unité d'acceptation de taux d'étanchéité d'huile (105e) configurée pour accepter une entrée d'un taux d'étanchéité d'huile représentant un rapport d'une quantité du lubrifiant en fonction d'une capacité du compartiment d'accueil d'huile intra-réducteur (30a), dans lequel
      l'unité de notification d'état d'endommagement (105a) est configurée pour notifier l'état d'endommagement du réducteur (40) correspondant au seuil lorsqu'une différence de couleur entre la couleur détectée par l'élément de réception de lumière de couleur (72) et une couleur prédéterminée atteint le seuil défini par l'unité de paramétrage de seuil (105b), et
      l'unité de paramétrage de seuil (105b) est adaptée pour définir le seuil en fonction du taux d'étanchéité d'huile dont l'entrée est acceptée par l'unité d'acceptation de taux d'étanchéité d'huile (105e).

2. Le dispositif de notification d'état d'endommagement de réducteur selon la revendication 1, comprenant en outre :

une unité d'acceptation de température de lubrifiant (105c) configurée pour accepter une entrée de température du lubrifiant, dans lequel l'unité de paramétrage de seuil (105b) est configurée pour définir le seuil en fonction de la température du lubrifiant dont l'entrée est acceptée par l'unité d'acceptation de température de lubrifiant (105c).

3. Le dispositif de notification d'état d'endommagement de réducteur selon la revendication 2, dans lequel
la machine comprend un capteur de température de lubrifiant (76) pour la détection de la température du lubrifiant, et
l'unité d'acceptation de température de lubrifiant (105c) est configurée pour accepter une entrée de température du lubrifiant détectée par le capteur de température de lubrifiant (76).

4. Le dispositif de notification d'état d'endommagement de réducteur selon la revendication 3, dans lequel
l'unité de paramétrage de seuil (105b) est configurée pour définir le seuil en fonction d'une valeur moyenne de la température du lubrifiant, sur une période de temps prédéterminée, dont l'entrée est acceptée par l'unité d'acceptation de température de lubrifiant (105c).

5. Le dispositif de notification d'état d'endommagement de réducteur selon la revendication 3, dans lequel
l'unité de notification d'état d'endommagement est configurée pour notifier l'état d'endommagement du réducteur (40) lorsqu'une quantité croissante d'une valeur moyenne de la température du lubrifiant sur une période de temps prédéterminée, dont l'entrée est acceptée par l'unité d'acceptation de température de lubrifiant (105c), est une valeur prédéterminée ou supérieure.

6. Le dispositif de notification d'état d'endommagement de réducteur selon une parmi les revendications 1 à 5, comprenant en outre
une unité de mémoire de correspondance type/capacité (104) configurée pour stocker une capacité du compartiment d'accueil d'huile intra-réducteur (30a) correspondant à un type du réducteur (40), dans lequel l'unité d'acceptation de taux d'étanchéité d'huile (105e) comprend une unité d'acceptation de type de réducteur (105f) configurée pour accepter une entrée du type du réducteur (40), et une unité d'acceptation de quantité de lubrifiant (105g) configurée pour accepter une entrée d'une quantité du lubrifiant,
l'unité d'acceptation de taux d'étanchéité d'huile (105e) accepte indirectement l'entrée du taux d'étanchéité d'huile en calculant le taux d'étanchéité d'huile en fonction de la capacité du compartiment d'accueil d'huile intra-réducteur (30a), qui est stockée dans l'unité de mémoire de correspondance type/capacité (104) en correspondance avec le type du réducteur (40) dont une entrée est acceptée par l'unité d'acceptation de type de réducteur (105f), et la quantité de lubrifiant dont l'entrée est acceptée par l'unité d'acceptation de quantité de lubrifiant (105g), et
l'unité de paramétrage de seuil (105b) est configurée pour définir le seuil en outre en fonction du type du réducteur (40) dont l'entrée est acceptée par l'unité d'acceptation de type de réducteur (105f).

7. Le dispositif de notification d'état d'endommagement de réducteur selon une parmi les revendications 1 à 5, comprenant en outre
une unité d'acceptation de compartiment d'accueil d'huile (105d) configurée pour accepter une entrée d'une capacité du compartiment d'accueil d'huile (30), dans lequel
l'unité de paramétrage de seuil (105b) est configurée pour définir le seuil en outre en fonction de la capacité du compartiment d'accueil d'huile (30) dont l'entrée est acceptée par l'unité d'acceptation de compartiment d'accueil d'huile (105d).

8. Le dispositif de notification d'état d'endommagement de réducteur selon une parmi les revendications 1 à 7, dans lequel le seuil comprend plusieurs niveaux de seuils correspondant à une probabilité de l'endommagement du réducteur (40).

9. Un système de machine avec une fonction de notification d'état d'endommagement de réducteur, comprenant :

le dispositif de notification d'état d'endommagement de réducteur selon une parmi les revendications 1 à 8 ; et
la machine à laquelle l'état d'endommagement du réducteur (40) est notifié par le dispositif de notification d'état d'endommagement de réducteur.

10. Un système de machine avec une fonction de notification d'état d'endommagement de réducteur, comprenant :

le dispositif de notification d'état d'endommagement de réducteur selon une parmi les revendications 3 à 5 ; et la machine à laquelle l'état d'endommagement du réducteur (40) est notifié par le dispositif de notification d'état d'endommagement de réducteur, dans lequel

le capteur de température de lubrifiant (76) est contenu dans le capteur de dégradation de lubrifiant (60).

11. Support qui enregistre un programme de notification d'état d'endommagement de réducteur (104a) pour faire fonctionner un ordinateur comme le dispositif de notification d'état d'endommagement de réducteur selon une parmi les revendications 1 à 8.

# FIG. 1

10 : MACHINE SYSTEM WITH REDUCER
DAMAGE-STATE NOTIFICATION FUNCTION

| INDUSTRIAL ROBOT | 20 |
|---|---|

11

NETWORK

| REDUCER DAMAGE-STATE NOTIFICATION APPARATUS | 100 |
|---|---|

# FIG. 2

# FIG. 3

# FIG. 4

# FIG. 5

# FIG. 6

(a)

(b)

# FIG. 7

## FIG. 8

100: REDUCER DAMAGE-STATE NOTIFICATION APPARATUS

105: CONTROL UNIT

104: MEMORY UNIT

105a
DAMAGE-STATE NOTIFICATION UNIT

105b
THRESHOLD SETTING UNIT

105c
LUBRICANT TEMPERATURE ACCEPTANCE UNIT

105d
INTRA-ARTICULAR PORTION OIL ACCOMMODATION SPACE

105e: OIL SEALING RATE ACCEPTANCE UNIT

105f
REDUCER TYPE ACCEPTANCE UNIT

105g
LUBRICANT AMOUNT ACCEPTANCE UNIT

101
OPERATION UNIT

102
DISPLAY UNIT

103
NETWORK COMMUNICATION UNIT

104a
REDUCER DAMAGE-STATE NOTIFICATION PROGRAM

104b
TYPE/CAPACITY CORRESPONDENCE TABLE

104c
THRESHOLD SETTING TABLE

104d
TABLE SELECTION TABLE

EP 2 829 864 B1

# FIG. 9

104b: TYPE/CAPACITY CORRESPONDENCE TABLE

| TYPE OF REDUCER | CAPACITY(ml) OF INTRA-REDUCER OIL-ACCOMMODATION SPACE |
|---|---|
| RV−XX1 | 50 |
| RV−XX2 | 100 |
| RV−XX3 | 150 |
| . | . |
| . | . |
| . | . |

# FIG. 10

104c: THRESHOLD SETTING TABLE

| TEMPERATURE OF LUBRICANT [°C] | OIL SEALING RATE [%] | | | | | | | | | |
|---|---|---|---|---|---|---|---|---|---|---|
| | 30 OR MORE AND LESS THAN 40 | 40 OR MORE AND LESS THAN 50 | 50 OR MORE AND LESS THAN 60 | 60 OR MORE AND LESS THAN 70 | 70 OR MORE AND LESS THAN 80 | 80 OR MORE AND LESS THAN 90 | 90 OR MORE AND LESS THAN 100 | 100 OR MORE AND LESS THAN 110 | 110 OR MORE AND LESS THAN 120 | 120 OR MORE |
| -10 OR MORE AND LESS THAN 0 | | | | | | | | | | |
| 0 OR MORE AND LESS THAN 10 | | | | | | | | | | |
| 10 OR MORE AND LESS THAN 20 | | | | | | | | | | |
| 20 OR MORE AND LESS THAN 30 | | | | | | | | | | |
| 30 OR MORE AND LESS THAN 40 | | | | | | | | | | |
| 40 OR MORE AND LESS THAN 50 | | | | | | 285 335 | | | 325 380 | |
| 50 OR MORE AND LESS THAN 60 | | | | | | | | | | |
| 60 OR MORE AND LESS THAN 70 | | | | | | 250 300 | | | 280 350 | |
| 70 OR MORE AND LESS THAN 80 | | | | | | | | | | |

EP 2 829 864 B1

# FIG. 11

104d: TABLE SELECTION TABLE

| | | CAPACITY (ml) OF INTRA-ARTICULAR PORTION OIL-ACCOMMODATION SPACE | | | |
|---|---|---|---|---|---|
| | | LESS THAN100 | 100 OR MORE AND LESS THAN 150 | 150 OR MORE AND LESS THAN 200 | · · · |
| TYPE OF REDUCER | RV−XX1 | TABLE 1 | TABLE 2 | TABLE 4 | · · · |
| | RV−XX2 | | TABLE 3 | TABLE 5 | · · · |
| | RV−XX3 | | | TABLE 6 | · · · |
| | · · · | · · · | · · · | · · · | · · · · |

# FIG. 12

(a)

(b)

(c)

## FIG. 13

```
                    ┌──────────────┐
                    │    START     │
                    └──────┬───────┘
                           │                              S201
                    ┌──────▼─────────────────────┐
                    │ DISPLAY INFORMATION INPUT  │
                    │         SCREEN             │
                    └──────┬─────────────────────┘
                           │                  S202
                       ╱───▼────╲                      ┌──────────┐
                      ╱    IS     ╲     NO              │          │
                     ╱ SETTING BUTTON╲──────────────────┘          │
                      ╲  PUSHED ?   ╱                              │
                       ╲──────────╱                               │
                           │ YES                                  │
                           │            S203                      │
                       ╱───▼────╲                                 │
                      ╱    IS     ╲    NO                          │
                     ╱ TYPE OF REDUCER╲────────────┐              │
                      ╲  NORMAL ?   ╱               │              │
                       ╲──────────╱                │              │
                           │ YES                   │              │
                    ┌──────▼─────────────┐  S204    │              │
                    │ STORE TYPE OF REDUCER│        │              │
                    └──────┬─────────────┘         │              │
                           │                       │              │
                       ╱───▼────╲        S205      │              │
                      ╱    IS     ╲                 │              │
                     ╱ CAPACITY OF ╲  NO            │              │
                    ╱INTRA-ARTICULAR PORTION╲───────┤              │
                    ╲OIL-ACCOMMODATION SPACE╱       │              │
                     ╲   NORMAL ?  ╱                │              │
                       ╲──────────╱                │              │
                           │ YES                   │              │
                    ┌──────▼───────────────┐ S206   │              │
                    │STORE CAPACITY OF INTRA-│      │              │
                    │ARTICULAR PORTION OIL-  │      │              │
                    │ACCOMMODATION SPACE     │      │              │
                    └──────┬───────────────┘       │              │
                           │          S207          │              │
                       ╱───▼────╲                   │              │
                      ╱    IS     ╲   NO             │              │
                     ╱ AMOUNT OF LUBRICANT╲─────────┤              │
                      ╲  NORMAL ?   ╱                │              │
                       ╲──────────╱                 │              │
                           │ YES    S208            │ S210          │
                    ┌──────▼───────────┐   ┌────────▼──────────┐   │
                    │STORE AMOUNT OF   │   │DISPLAY ERROR SCREEN│───┘
                    │   LUBRICANT      │   └───────────────────┘
                    └──────┬───────────┘
                           │        S209
                    ┌──────▼───────────┐
                    │STORE OIL SEALING │
                    │     RATE         │
                    └──────┬───────────┘
                           │
                         ( 1 )
```

# FIG. 14

①

S221
DETERMINE THRESHOLD
SETTING TABLE

S222
OBTAIN TEMPERATURE OF LUBRICANT

S223
CALCULATE AVERAGE VALUE
OF TEMPERATURE DURING
PREDETERMINED TIME PERIOD

S224
IS
INCREASING AMOUNT OF
AVERAGE VALUE OF TEMPERATURE
PREDETERMINED VALUE OR
MORE ?

YES

NO

S225
NOTIFICATION FOR WARNING
THAT REDUCER IS DAMAGED
WITH HIGH PROBABILITY

S226
SET THRESHOLD

S227
OBTAIN COLOR DIFFERENCE ΔE

S228
COLOR
DIFFERENCE ΔE ≤ WARNING
THRESHOLD ?

NO

YES

S229
COLOR
DIFFERENCE ΔE ≤ CHECK/REPAIR
THRESHOLD ?

NO

YES

S231
NOTIFICATION FOR PROMOTING
CHECKING OR REPAIRING OF
REDUCER

S230
NOTIFICATION FOR WARNING
THAT REDUCER MAY BE
DAMAGED

# FIG. 15

TYPE OF REDUCER

CAPACITY OF INTRA-ARTICULAR
PORTION OIL ACCOMMODATION
SPACE

AMOUNT OF LUBRICANT

ml

ml

SET

111

112

113

114

# FIG. 16

REDUCER IS DAMAGED WITH
HIGH PROBABILITY

(a)

REDUCER MAY BE DAMAGED

(b)

REDUCER IS DAMAGED WITH
EXTREMELY HIGH PROBABILITY

PLEASE CHECK OR REPAIR

(c)

# FIG. 17

(a)

(b)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7146233 A
- JP 10104160 A

- US 2008024761 A1

**Non-patent literature cited in the description**

- METHOD OF DISCRIMINATING HUE OF CONTAMINANT WITHIN LUBRICANT. **TOMOHIKO YAMAGUCHI.** Faculty of Engineering, Research Report. Fukui University, March 2003, vol. 51, 81-88

- **TOMOMI HONDA.** DETERIORATION DIAGNOSIS OF LUBRICANT •INSPECTION TECHNOLOGY. *Journal of Japan Society for Precision Engineering,* 2009, vol. 75 (3), 359-362